# EUROPEAN PATENT APPLICATION

(11) **EP 2 080 511 A2**
(43) Date of publication of application: **22.07.2009**
(21) Application number: 09152598.0
(22) Date of filing: 22.08.2001
(51) Int. Cl.: A61K 9/14, A61K 9/22, A61K 9/50, A61K 47/42, C07K 1/02, C07K 1/13

(54) **Active agent delivery systems and methods for protecting and administering active agents**

(30) Priority: 22.08.2000 US 642820; 14.11.2000 US 247622 P; 14.11.2000 US 247621 P; 14.11.2000 US 247620 P; 14.11.2000 US 247595 P; 14.11.2000 US 247594 P; 14.11.2000 US 247635 P; 14.11.2000 US 247634 P; 14.11.2000 US 247606 P; 14.11.2000 US 247607 P; 14.11.2000 US 247608 P; 14.11.2000 US 247609 P; 14.11.2000 US 247610 P; 14.11.2000 US 247611 P; 14.11.2000 US 247702 P; 14.11.2000 US 247701 P; 14.11.2000 US 247700 P; 14.11.2000 US 247699 P; 14.11.2000 US 247698 P; 14.11.2000 US 247807 P; 14.11.2000 US 247833 P; 14.11.2000 US 247832 P; 14.11.2000 US 247927 P; 14.11.2000 US 247926 P; 14.11.2000 US 247930 P; 14.11.2000 US 247929 P; 14.11.2000 US 247928 P; 14.11.2000 US 247797 P; 14.11.2000 US 247805 P; 14.11.2000 US 247804 P; 14.11.2000 US 247803 P; 14.11.2000 US 247802 P; 14.11.2000 US 247801 P; 14.11.2000 US 247800 P; 14.11.2000 US 247799 P; 14.11.2000 US 247798 P; 14.11.2000 US 247561 P; 14.11.2000 US 247560 P; 14.11.2000 US 247559 P; 14.11.2000 US 247558 P; 14.11.2000 US 247556 P; 14.11.2000 US 247612 P; 14.11.2000 US 247613 P; 14.11.2000 US 247614 P; 14.11.2000 US 247615 P; 14.11.2000 US 247616 P; 14.11.2000 US 247617 P; 14.11.2000 US 247633 P; 14.11.2000 US 247632 P; 14.11.2000 US 247631 P; 14.11.2000 US 247630 P; 14.11.2000 US 247629 P; 14.11.2000 US 247628 P; 14.11.2000 US 247627 P; 14.11.2000 US 247626 P; 14.11.2000 US 247625 P; 14.11.2000 US 247624 P; 14.11.2000 US 247806 P; 14.11.2000 US 247563 P; 14.11.2000 US 247795 P; 14.11.2000 US 247794 P; 14.11.2000 US 247793 P; 14.11.2000 US 247792 P; 14.11.2000 US 247791 P; 14.11.2000 US 247790 P; 14.11.2000 US 247789 P; 14.11.2000 US 247788 P; 14.11.2000 US 247787 P; 14.11.2000 US 247786 P; 14.11.2000 US 247785 P; 14.11.2000 US 247784 P; 14.11.2000 US 247783 P; 14.11.2000 US 247782 P; 14.11.2000 US 247781 P; 14.11.2000 US 247780 P; 14.11.2000 US 247779 P; 14.11.2000 US 247778 P; 14.11.2000 US 247777 P; 14.11.2000 US 247776 P; 14.11.2000 US 247775 P; 14.11.2000 US 247774 P; 14.11.2000 US 247773 P; 14.11.2000 US 247772 P; 14.11.2000 US 247770 P; 14.11.2000 US 247769 P; 14.11.2000 US 247768 P; 14.11.2000 US 247767 P; 14.11.2000 US 247766 P; 14.11.2000 US 247871 P; 14.11.2000 US 247872 P; 14.11.2000 US 247873 P; 14.11.2000 US 247874 P; 14.11.2000 US 247875 P; 14.11.2000 US 247981 P; 14.11.2000 US 247982 P; 14.11.2000 US 247983 P; 14.11.2000 US 247984 P; 14.11.2000 US 247745 P; 14.11.2000 US 247744 P; 14.11.2000 US 247743 P; 14.11.2000 US 247742 P; 14.11.2000 US 247623 P; 14.11.2000 US 247985 P; 14.11.2000 US 247840 P; 14.11.2000 US 247839 P; 14.11.2000 US 247838 P; 14.11.2000 US 247837 P; 14.11.2000 US 247836 P; 14.11.2000 US 247889 P; 14.11.2000 US 247890 P; 14.11.2000 US 247891 P; 14.11.2000 US 247892 P; 14.11.2000 US 247893 P; 14.11.2000 US 247741 P; 14.11.2000 US 247740 P; 14.11.2000 US 247739 P; 14.11.2000 US 247738 P; 14.11.2000 US 247737 P; 14.11.2000 US 247736 P; 14.11.2000 US 247735 P; 14.11.2000 US 247734 P; 14.11.2000 US 247733 P; 14.11.2000 US 247732 P; 14.11.2000 US 247731 P; 14.11.2000 US 247730 P; 14.11.2000 US 247728 P; 14.11.2000 US 247729 P; 14.11.2000 US 247727 P; 14.11.2000 US 247726 P; 14.11.2000 US 247761 P; 14.11.2000 US 247760 P; 14.11.2000 US 247759 P; 14.11.2000 US 247758 P; 14.11.2000 US 247757 P; 14.11.2000 US 247756 P; 14.11.2000 US 247765 P; 14.11.2000 US 247764 P; 14.11.2000 US 247763 P; 14.11.2000 US 247762 P; 14.11.2000 US 247755 P; 14.11.2000 US 247746 P; 14.11.2000 US 247725 P; 14.11.2000 US 247724 P; 14.11.2000 US 247723 P; 14.11.2000 US 247722 P; 14.11.2000 US 247721 P; 14.11.2000 US 247720 P; 14.11.2000 US 247719 P; 14.11.2000 US 247718 P; 14.11.2000 US 247717 P; 14.11.2000 US 247716 P; 14.11.2000 US 247754 P; 14.11.2000 US 247753 P; 14.11.2000 US 247752 P; 14.11.2000 US 247751 P; 14.11.2000 US 247750 P; 14.11.2000 US 247749 P; 14.11.2000 US 247748 P; 14.11.2000 US 247747 P; 14.11.2000 US 247796 P; 14.11.2000 US 247815 P; 14.11.2000 US 247814 P; 14.11.2000 US 247813 P; 14.11.2000 US 247812 P; 14.11.2000 US 247811 P; 14.11.2000 US 247810 P; 14.11.2000 US 247809 P; 14.11.2000 US 247808 P; 14.11.2000 US 247885 P; 14.11.2000 US 247884 P; 14.11.2000 US 247883 P; 14.11.2000 US 247882 P; 14.11.2000 US 247881 P; 14.11.2000 US 247880 P; 14.11.2000 US 247879 P; 14.11.2000 US 247878 P; 14.11.2000 US 247826 P; 14.11.2000 US 247835 P; 14.11.2000 US 247834 P; 14.11.2000 US 247897 P; 14.11.2000 US 247896 P; 14.11.2000 US 247895 P; 14.11.2000 US 247894 P; 14.11.2000 US 247901 P; 14.11.2000 US 247900 P; 14.11.2000 US 247899 P; 14.11.2000 US 247898 P; 14.11.2000 US 247903 P; 14.11.2000 US 247902 P; 14.11.2000 US 247919 P; 14.11.2000 US 247918 P; 14.11.2000 US 247917 P; 14.11.2000 US 247916 P; 14.11.2000 US 247915 P; 14.11.2000 US 247914 P; 14.11.2000 US 247913 P; 14.11.2000 US 247912 P; 14.11.2000 US 247911 P; 14.11.2000 US 247910 P; 14.11.2000 US 247877 P; 14.11.2000 US 247876 P; 14.11.2000 US 247707 P; 14.11.2000 US 247706 P; 14.11.2000 US 247705 P; 14.11.2000 US 247704 P; 14.11.2000 US 247703 P; 14.11.2000 US 247692 P; 14.11.2000 US 247691 P; 14.11.2000 US 247690 P; 14.11.2000 US 247689 P; 14.11.2000 US 247688 P; 14.11.2000 US 247687 P; 14.11.2000 US 247686 P; 14.11.2000 US 247685 P; 14.11.2000 US 247684 P; 14.11.2000 US 247683 P; 14.11.2000 US 247694 P; 14.11.2000 US 247693 P; 14.11.2000 US 247712 P; 14.11.2000 US 247711 P; 14.11.2000 US 247710 P; 14.11.2000 US 247709 P; 14.11.2000 US 247708 P; 14.11.2000 US 247697 P; 14.11.2000 US 247696 P; 14.11.2000 US 247695 P; 14.11.2000 US 247565 P; 14.11.2000 US 247564 P; 14.11.2000 US 247545 P; 14.11.2000 US 247546 P; 14.11.2000 US 247547 P; 14.11.2000 US 247548 P; 14.11.2000 US 247568 P; 14.11.2000 US 247570 P; 14.11.2000 US 247580 P; 14.11.2000 US 247555 P; 14.11.2000 US 247554 P; 14.11.2000 US 247553 P; 14.11.2000 US 247552 P; 14.11.2000 US 247551 P; 14.11.2000 US 247682 P; 14.11.2000 US 247681 P; 14.11.2000 US 247680 P; 14.11.2000 US 247679 P; 14.11.2000 US 247678 P; 14.11.2000 US 247677 P; 14.11.2000 US 247676 P; 14.11.2000 US 247655 P; 14.11.2000 US 247645 P; 14.11.2000 US 247656 P; 14.11.2000 US 247771 P; 16.11.2000 US 248607 P; 16.11.2000 US 248611 P; 16.11.2000 US 248609 P; 16.11.2000 US 248608 P; 16.11.2000 US 248606 P; 16.11.2000 US 248604 P; 16.11.2000 US 248603 P; 16.11.2000 US 248601 P; 16.11.2000 US 248600 P; 16.11.2000 US 248712 P; 16.11.2000 US 248711 P; 16.11.2000 US 248709 P; 16.11.2000 US 248708 P; 16.11.2000 US 248707 P; 16.11.2000 US 248706 P; 16.11.2000 US 248705 P; 16.11.2000 US 248704 P; 16.11.2000 US 248703 P; 16.11.2000 US 248702 P; 16.11.2000 US 248701 P; 16.11.2000 US 248700 P; 16.11.2000 US 248699 P; 16.11.2000 US 248698 P; 16.11.2000 US 248697 P; 16.11.2000 US 248696 P; 16.11.2000 US 248695 P; 16.11.2000 US 248694 P; 16.11.2000 US 248693 P; 16.11.2000 US 248692 P; 16.11.2000 US 248691 P; 16.11.2000 US 248710 P; 16.11.2000 US 248689 P; 16.11.2000 US 248688 P; 16.11.2000 US 248686 P; 16.11.2000 US 248720 P; 16.11.2000 US 248719 P; 16.11.2000 US 248718 P; 16.11.2000 US 248716 P; 16.11.2000 US 248715 P; 16.11.2000 US 248714 P; 16.11.2000 US 248713 P; 16.11.2000 US 248536 P; 16.11.2000 US 248535 P; 16.11.2000 US 248733 P; 16.11.2000 US 248732 P; 16.11.2000 US 248731 P; 16.11.2000 US 248730 P; 16.11.2000 US 248729 P; 16.11.2000 US 248728 P; 16.11.2000 US 248727 P; 16.11.2000 US 248726 P; 16.11.2000 US 248725 P; 16.11.2000 US 248724 P; 16.11.2000 US 248723 P; 16.11.2000 US 248722 P; 16.11.2000 US 248721 P; 16.11.2000 US 248540 P; 16.11.2000 US 248539 P; 16.11.2000 US 248538 P; 16.11.2000 US 248537 P; 16.11.2000 US 248533 P; 16.11.2000 US 248532 P; 16.11.2000 US 248531 P; 16.11.2000 US 248530 P; 16.11.2000 US 248529 P; 16.11.2000 US 248528 P; 16.11.2000 US 248527 P; 16.11.2000 US 248526 P; 16.11.2000 US 248525 P; 16.11.2000 US 248524 P; 16.11.2000 US 248670 P; 16.11.2000 US 248789 P; 16.11.2000 US 248599 P; 16.11.2000 US 248745 P; 16.11.2000 US 248746 P; 16.11.2000 US 248747 P; 16.11.2000 US 248748 P; 16.11.2000 US 248744 P; 16.11.2000 US 248743 P; 16.11.2000 US 248756 P; 16.11.2000 US 248602 P; 16.11.2000 US 248598 P; 16.11.2000 US 248597 P; 16.11.2000 US 248596 P; 16.11.2000 US 248595 P; 16.11.2000 US 248594 P; 16.11.2000 US 248858 P; 16.11.2000 US 248857 P; 16.11.2000 US 248856 P; 16.11.2000 US 248855 P; 16.11.2000 US 248854 P; 16.11.2000 US 248853 P; 16.11.2000 US 248852 P; 16.11.2000 US 248851 P; 16.11.2000 US 248850 P; 16.11.2000 US 248849 P; 16.11.2000 US 248848 P; 16.11.2000 US 248792 P; 16.11.2000 US 248790 P; 16.11.2000 US 248669 P; 16.11.2000 US 248668 P; 16.11.2000 US 248667 P; 16.11.2000 US 248666 P; 16.11.2000 US 248665 P; 16.11.2000 US 248664 P; 16.11.2000 US 248793 P; 16.11.2000 US 248791 P; 16.11.2000 US 248684 P; 16.11.2000 US 248683 P; 16.11.2000 US 248682 P; 16.11.2000 US 248681 P; 16.11.2000 US 248680 P; 16.11.2000 US 248671 P; 16.11.2000 US 248679 P; 16.11.2000 US 248675 P; 16.11.2000 US 248676 P; 16.11.2000 US 248677 P; 16.11.2000 US 248678 P; 16.11.2000 US 248673 P; 16.11.2000 US 248674 P; 16.11.2000 US 248672 P; 16.11.2000 US 248784 P; 16.11.2000 US 248785 P; 16.11.2000 US 248786 P; 16.11.2000 US 248775 P; 16.11.2000 US 248773 P; 16.11.2000 US 248766 P; 16.11.2000 US 248765 P; 16.11.2000 US 248833 P; 16.11.2000 US 248783 P; 16.11.2000 US 248781 P; 16.11.2000 US 248780 P; 16.11.2000 US 248778 P; 16.11.2000 US 248767 P; 16.11.2000 US 248787 P; 16.11.2000 US 248774 P; 16.11.2000 US 248764 P; 16.11.2000 US 248782 P; 16.11.2000 US 248779 P; 16.11.2000 US 248685 P; 16.11.2000 US 248772 P; 16.11.2000 US 248771 P; 16.11.2000 US 248777 P; 16.11.2000 US 248776 P; 16.11.2000 US 248770 P; 16.11.2000 US 248768 P; 16.11.2000 US 248769 P; 16.11.2000 US 248796 P; 16.11.2000 US 248797 P; 16.11.2000 US 248795 P; 16.11.2000 US 248794 P; 16.11.2000 US 248663 P; 16.11.2000 US 248662 P; 16.11.2000 US 248660 P; 16.11.2000 US 248659 P; 16.11.2000 US 248658 P; 16.11.2000 US 248656 P; 16.11.2000 US 248654 P; 16.11.2000 US 248653 P; 16.11.2000 US 248651 P; 16.11.2000 US 248650 P; 16.11.2000 US 248648 P; 16.11.2000 US 248647 P; 16.11.2000 US 248645 P; 16.11.2000 US 248643 P; 16.11.2000 US 248642 P; 16.11.2000 US 248640 P; 16.11.2000 US 248637 P; 16.11.2000 US 248636 P; 16.11.2000 US 248634 P; 16.11.2000 US 248632 P; 16.11.2000 US 248631 P; 16.11.2000 US 248630 P; 16.11.2000 US 248629 P; 16.11.2000 US 248627 P; 16.11.2000 US 248625 P; 16.11.2000 US 248763 P; 16.11.2000 US 248761 P; 16.11.2000 US 248759 P; 16.11.2000 US 248757 P; 16.11.2000 US 248754 P; 16.11.2000 US 248753 P; 16.11.2000 US 248749 P; 16.11.2000 US 248616 P; 16.11.2000 US 248615 P; 16.11.2000 US 248614 P; 16.11.2000 US 248613 P; 16.11.2000 US 248612 P; 16.11.2000 US 248605 P; 16.11.2000 US 248610 P; 16.11.2000 US 248661 P; 16.11.2000 US 248657 P; 16.11.2000 US 248655 P; 16.11.2000 US 248652 P; 16.11.2000 US 248649 P; 16.11.2000 US 248646 P; 16.11.2000 US 248644 P; 16.11.2000 US 248641 P; 16.11.2000 US 248639 P; 16.11.2000 US 248638 P; 16.11.2000 US 248635 P; 16.11.2000 US 248633 P; 16.11.2000 US 248628 P; 16.11.2000 US 248626 P; 16.11.2000 US 248624 P; 16.11.2000 US 248762 P; 16.11.2000 US 248760 P; 16.11.2000 US 248758 P; 16.11.2000 US 248755 P; 16.11.2000 US 248752 P; 16.11.2000 US 248751 P; 16.11.2000 US 248750 P; 16.11.2000 US 248742 P; 16.11.2000 US 248741 P; 16.11.2000 US 248740 P; 16.11.2000 US 248739 P; 16.11.2000 US 248736 P; 16.11.2000 US 248735 P; 16.11.2000 US 248734 P; 16.11.2000 US 248623 P; 16.11.2000 US 248622 P; 16.11.2000 US 248621 P; 16.11.2000 US 248738 P; 16.11.2000 US 248737 P; 16.11.2000 US 248620 P; 16.11.2000 US 248619 P; 16.11.2000 US 248618 P; 16.11.2000 US 248617 P; 16.11.2000 US 248687 P; 16.11.2000 US 248690 P; 16.11.2000 US 248717 P
(62) Divisional of application: 01966056.2
(71) Applicant: Shire LLC, Florence, KY 41042 (US)
(72) Inventor: Piccariello, Thomas, Blacksburg, VA 24060 (US); Olon, Lawrence P, Bristol, TN 37620-2948 (US); Kirk, Randall J, Radford, VA 24060 (US)
(74) Representative: Harrison Goddard Foote

(57) **Abstract**

A composition comprising a polypeptide and an active agent covalently attached to the polypeptide. Also provided is a method for delivery of an active agent to a patient comprising administering to the patient a composition comprising a polypeptide and an active agent covalently attached to the polypeptide. Also provided is a method for protecting an active agent from degradation comprising covalently attaching the active agent to a polypeptide. Also provided is a method for controlling release of an active agent from a composition comprising covalently attaching the active agent to the polypeptide.

## Description

### Field of the Invention

The present invention relates to active agent delivery systems and, more specifically, to compositions that comprise polypeptides covalently attached to active agents and methods for protecting and administering active agents.

### Background of the Invention

Active agent delivery systems are often critical for the effective delivery of a biologically active agent (active agent) to the appropriate target. The importance of these systems becomes magnified when patient compliance and active agent stability are taken under consideration. For instance, one would expect patient compliance to increase markedly if an active agent is administered orally in lieu of an injection or another invasive technique. Increasing the stability of the active agent, such as prolonging shelf life or survival in the stomach, will assure dosage reproducibility and perhaps even reduce the number of dosages required which could improve patient compliance.

Absorption of an orally administered active agent is often blocked by the harshly acidic stomach milieu, powerful digestive enzymes in the GI tract, permeability of cellular membranes and transport across lipid bilayers. Incorporating adjuvants such as resorcinol, surfactants, polyethylene glycol (PEG) or bile acids enhance permeability of cellular membranes. Microencapsulating active agents using protenoid microspheres, liposomes or polysaccharides have been effective in abating enzyme degradation of the active agent. Enzyme inhibiting adjuvants have also been used to prevent enzyme degradation. Enteric coatings have been used as a protector of pharmaceuticals in the stomach.

Active agent delivery systems also provide the ability to control the release of the active agent. For example, formulating diazepam with a copolymer of glutamic acid and aspartic acid enables a sustained release of the active agent. As another example, copolymers of lactic acid and glutaric acid are used to provide timed release of human growth hormone. A wide range of pharmaceuticals purportedly provide sustained release through microencapsulation of the active agent in amides of dicarboxylic acids, modified amino acids or thermally condensed amino acids. Slow release rendering additives can also be intermixed with a large array of active agents in tablet formulations.

Each of these technologies imparts enhanced stability and time-release properties to active agent substances. Unfortunately, these technologies suffer from several shortcomings. Incorporation of the active agent is often dependent on diffusion into the microencapsulating matrix, which may not be quantitative and may complicate dosage reproducibility. In addition, encapsulated drugs rely on diffusion out of the matrix, which is highly dependant on the water solubility of the active agent. Conversely, water-soluble microspheres swell by an infinite degree and, unfortunately, may release the active agent in bursts with little active agent available for sustained release. Furthermore, in some technologies, control of the degradation process required for active agent release is unreliable. For example, an enterically coated active agent depends on pH to release the active agent and, as such, is difficult to control the rate of release.

In the past, use has been made of amino acid side chains of polypeptides as pendant groups to which active agents can be attached. These technologies typically require the use of spacer groups between the amino acid pendant group and the active agent. The peptide-drug conjugates of this class of drug delivery system rely on enzymes in the bloodstream for the release of the drug and, as such, are not used for oral administration. Examples of timed and targeted release of injectable or subcutaneous pharmaceuticals include: linking of norethindrone, via a hydroxypropyl spacer, to the gamma, carboxylate of polyglutamic acid; and linking of nitrogen mustard, via a peptide spacer, to the gamma carbamide of polyglutamine. Dexamethasone has been covalently attached directly to the beta carboxylate of polyaspartic acid without a spacer group. This prodrug formulation was designed as a colon-specific drug delivery system where the drug is released by bacterial hydrolytic enzymes residing in the large intestines. The released dexamethasone active agent, in turn, was targeted to treat large bowel disorders and was not intended to be absorbed into the bloodstream. Yet another technology combines the advantages of covalent drug attachment with liposome formation where the active ingredient is attached to highly ordered lipid films (known as HARs) via a peptide linker. Thus, there has been no drug delivery system, heretofore reported, that incorporates the concept of attaching an active ingredient to a polypeptide pendant group with its targeted delivery into the bloodstream via oral administration.

It is also important to control the molecular weight, molecular size and particle size of the active agent delivery system. Variable molecular weights have unpredictable diffusion rates and pharmacokinetics. High molecular weight carriers are digested slowly or late, as in the case of naproxen-linked dextran, which is digested almost exclusively in the colon by bacterial enzymes. High molecular weight microspheres usually have high moisture content which may present a problem with water labile active ingredients. Particle size not only becomes a problem with injectable drugs, as in the HAR application, but absorption through the brush-border membrane of the intestines is limited to less than 5 microns.

### Summary of the Invention

The present invention provides covalent attachment of active agents to a polymer of peptides or amino acids. The invention is distinguished from the above mentioned technologies by virtue of covalently attaching the active agent, which includes, for example, pharmaceutical drugs and nutrients, to the N-terminus, the C-terminus or directly to the amino acid side chain of an oligopeptide or polypeptide, also referred to herein as a carrier peptide. In certain applications, the polypeptide will stabilize the active agent, primarily in the stomach, through conformational protection. In these applications, delivery of the active agent is controlled, in part, by the kinetics of unfolding of the carrier peptide. Upon entry into the upper intestinal tract, indigenous enzymes release the active ingredient for absorption by the body by selectively hydrolyzing the peptide bonds of the carrier peptide. This enzymatic action introduces a second order sustained release mechanism.

The invention provides a composition comprising a polypeptide and an active agent covalently attached to the polypeptide. Preferably, the polypeptide is (i) an oligopeptide, (ii) a homopolymer of one of the twenty naturally occurring amino acids (L or D isomers), or an isomer, analogue, or derivative thereof, (iii) a heteropolymer of two or more naturally occurring amino acids (L or D isomers), or an isomer, analogue, or derivative thereof, (iv) a homopolymer of a synthetic amino acid, (v) a heteropolymer of two or more synthetic amino acids or (vi) a heteropolymer of one or more naturally occurring amino acids and one or more synthetic amino acids.

The active agent preferably is covalently attached to a side chain, the N-terminus or the C-terminus of the polypeptide. In a preferred embodiment, the active agent is a carboxylic acid and is covalently attached to the N-terminus of the polypeptide. In another preferred embodiment, the active agent is an amine and is covalently attached to the C-terminus of the polypeptide. In another preferred embodiment, the active agent is an alcohol and is covalently attached to the C-terminus of the polypeptide. In yet another preferred embodiment, the active agent is an alcohol and is covalently attached to the N-terminus of the polypeptide.

The composition of the invention can also include one or more of a microencapsulating agent, an adjuvant and a pharmaceutically acceptable excipient. The microencapsulating agent can be selected from polyethylene glycol (PEG), an amino acid, a sugar and a salt. When an adjuvant is included in the composition, the adjuvant preferably activates an intestinal transporter.

Preferably, the composition of the invention is in the form of an ingestable tablet, an intravenous preparation or an oral suspension. The active agent can be conformationally protected by folding of the polypeptide about the active agent. In another embodiment, the polypeptide is capable of releasing the active agent from the composition in a pH-dependent manner.

The invention also provides a method for protecting an active agent from degradation comprising covalently attaching the active agent to a polypeptide.

The invention also provides a method for controlling release of an active agent from a composition wherein the composition comprises a polypeptide, the method comprising covalently attaching the active agent to the polypeptide.

The invention also provides a method for delivering an active agent to a patient, the patient being a human or a non-human animal, comprising administering to the patient a composition comprising a polypeptide and an active agent covalently attached to the polypeptide. In a preferred embodiment, the active agent is released from the composition by an enzyme-catalyzed release. In another preferred embodiment, the active agent is released in a time-dependent manner based on the pharmacokinetics of the enzyme-catalyzed release. In another preferred embodiment, the composition further comprises a microencapsulating agent and the active agent is released from the composition by dissolution of the microencapsulating agent. In another preferred embodiment, the active agent is released from the composition by a pH-dependent unfolding of the polypeptide. In another preferred embodiment, the active agent is released from the composition in a sustained release. In yet another preferred embodiment, the composition further comprises an adjuvant covalently attached to the polypeptide and release of the adjuvant from the composition is controlled by the polypeptide. The adjuvant can be microencapsulated into a carrier peptide-drug conjugate for biphasic release of active ingredients.

The invention also provides a method for preparing a composition comprising a polypeptide and an active agent covalently attached to the polypeptide. The method comprises the steps of:
(a) attaching the active agent to a side chain of an amino acid to form an active agent/amino acid complex;
(b) forming an active agent/amino acid complex N-carboxyanhydride (NCA) from the active agent/amino acid complex; and
(c) polymerizing the active agent/amino acid complex N-carboxyanhydride (NCA).

In a preferred embodiment, the active agent is a pharmaceutical agent or an adjuvant. In another preferred embodiment, steps (a) and (b) are repeated prior to step (c) with a second active agent. When steps (a) and (b) are repeated prior to step (c) with a second agent, the active agent and second active agent can be copolymerized in step (c). In another preferred embodiment, the amino acid is glutamic acid and the active agent is released from the glutamic acid as a dimer upon a hydrolysis of the polypeptide and wherein the active agent is released from the glutamic acid by coincident intramolecular transamination. In another preferred embodiment, the glutamic acid is replaced by an amino acid selected from the group consisting of aspartic acid, arginine, asparagine, cysteine, lysine, threonine, and serine, and wherein the active agent is attached to the side chain of the amino acid to form an amide, a thioester, an ester, an ether, a urethane, a carbonate, an anhydride or a carbamate. In yet another preferred embodiment, the glutamic acid is replaced by a synthetic amino acid with a pendant group comprising an amine, an alcohol, a sulfhydryl, an amide, a urea, or an acid functionality.

It is to be understood that both the foregoing general description and the following detailed description are exemplary, but are not restrictive, of the invention.

### BRIEF DESCRIPTION OF THE DRAWINGS

The invention is best understood from the following detailed description when read in connection with the accompanying drawing. Included in the drawing are the following figures.
Fig. 1 illustrates an acid active agent/N-terminus scheme of the invention.
Fig. 2 illustrates an amine active agent/C-terminus scheme of the invention.
Fig. 3 illustrates an alcohol active agent/N-terminus scheme of the invention.
Fig. 4 illustrates an alcohol active agent/glutamic acid dimer preparation and conjugation scheme of the invention.
Fig. 5 illustrates a mechanism of alcohol active agent from glutamic acid dimer scheme.
Fig. 6 illustrates the in situ digestion of polythroid in intestinal epithelial cell cultures.
Fig. 7 illustrates basolateral T4 concentrations.
Fig. 8 illustrates the polythroid concentration of basal versus basolateral.
Fig. 9 illustrates T4 analysis in gastric simulator versus intestinal simulator.
Fig. 10 illustrates T3 analysis in gastric simulator versus intestinal simulator.

### DETAILED DESCRIPTION OF THE INVENTION

The present invention provides several benefits for active agent delivery. First, the invention can stabilize the active agent and prevent digestion in the stomach. In addition, the pharmacologic effect can be prolonged by delayed release of the active agent. Furthermore, active agents can be combined to produce synergistic effects. Also, absorption of the active agent in the intestinal tract can be enhanced. The invention also allows targeted delivery of active agents to specifics sites of action.

The composition of the invention comprises a polypeptide and an active agent covalently attached to the polypeptide. Acive agents may be selected from the list in TABLE 1, either alone or in combination with other agents on the list.

**TABLE 1**

| |
|---|
| abacavir sulfate |
| abarelix |
| acarbose |
| Acetaminophen |
| Acetaminophen; Codeine phosphate |
| Acetaminophen; Propoxyphene napsylate |
| Acetylsalicylic acid |
| |
| Acitretin |
| activated protein C |
| Acyclovir |
| adefovir dipivoxil |
| adenosine |
| Adrenocorticotrophic hormone |
| |
| Albuterol |
| alendronate sodium |
| |
| Allopurinal |
| alpha 1 proteinase inhibitor |
| Alprazalom |
| alprostadil |
| altinicline |
| amifostine |
| Amiodarone |
| |
| Amitriptyline HCL |
| amlodipine besylate |
| amlodipine besylate; benazepril hcl |
| |
| Amoxicillin |
| amoxicillin; clavulanate potassium |
| amprenavir |
| anagrelide hydrochloride |
| anaritide |
| anastrozole |
| antisense oligonucleotide |
| aripiprazole |
| Astemizole |
| Atenolol |
| atorvastatin calcium |
| atovaquone |
| avasimibe |
| Azathioprine |
| azelastine hydrochloride |
| Azithromycin dihydrate |
| Baclofen |
| befloxatone |
| bonazepril hydrochloride |
| Benzatropine Mesylate |
| Betamethasone |
| bicalutamide |
| Bisoprolol/Hydrochlorothiazide |
| bosentan |
| Bromocriptine |
| Bupropion hydrochloride |
| Buspirone |
| Butorphanol tartrate |
| cabergoline |
| caffiene |
| calcitriol |
| candesartan cilexetil |
| candoxatril |
| capecitabine |
| Captopril |
| carbamazepine |
| Carbidopa/Levodopa |
| carboplatin |
| Carisoprodol |
| carvedilol |
| |
| caspofungin |
| Cefaclor |
| Cefadroxil; Cefadroxil hemihydrate |
| Cefazolin sodium |
| Cefdinir |
| Cefixime |
| 1555; 1555U88 |
| Cefotaxime sodium |
| Cefotetan disodium |
| Cefoxitin sodium |
| Cefpodoxime proxetil |
| Cefprozil |
| |
| Ceftazidime |
| Ceftibuten dihydrate |
| 264W94 |
| Cefuroxime axetil |
| |
| Cefuroxime sodium |
| celecoxib |
| |
| Cephalexin |
| cerivastatin sodium |
| cetirizine hydrochloride |
| |
| Chlorazepate Depot |
| Chlordiazepoxide |
| ciclesonide |
| cilansetron |
| Cilastatin sodium; Imipenem |
| |
| cilomilast |
| Cimetidine |
| ciprofloxacin |
| |
| cisapride |
| |
| cisatracurium besylate |
| cisplatin |
| citalopram hydrobromide |
| clarithromycin |
| Clomipramine |
| Clonazepam |
| Clonidine HCL |
| clopidogrel bisulfate |
| |
| 4030W92 |
| clorpheniramine tannate |
| |
| Clozapine |
| |
| Colestipol HCL |
| conivaptan |
| Cyclobenzaprine HCL |
| Cyclophosphamide |
| Cyclosporine |
| dalteparin sodium |
| dapitant |
| desmopressin acetate |
| Desogestrel; ethinyl estradiol |
| Dextroamphetamine sulfate |
| dextromethorphan |
| Diazepam |
| ABT 594 |
| Diclofenac sodium |
| diclofenac sodium, misoprostol |
| |
| Dicyclomine HCL |
| didanosine |
| Digoxin |
| |
| diltiazem hydrochloride |
| Dipyridamole |
| |
| divalproex sodium |
| d-methylphenidate |
| dolasetron mesylate monohydrate |
| donepezil hydrochloride |
| |
| Dopamine/D5W |
| Doxazosin |
| doxorubicin hydrochloride |
| duloxetine |
| dutasteride |
| ecadotril |
| ecopipam |
| edodekin alfa (Interleukin-12) |
| efavirenz |
| |
| ABT 773 |
| emivirine |
| Enalapril |
| enapril maleate, hydrochlorothiazide |
| eniluracil |
| enoxaparin sodium |
| |
| epoetin alfa recombinant |
| |
| eptifibatide |
| Ergotamine Tartrate |
| Erythromycin |
| ALT 711 |
| esatenolol |
| Esterified estrogens; |
| ,Methyltestosterone |
| Estrogens, conjugated |
| Estrogens, conjugated; |
| medroxyprogesterone acetate |
| Estropipate |
| etanercept |
| ethinyl estradiol/norethindrone |
| |
| BMS CW189921 |
| Ethinyl estradiol; Ethynodiol |
| diacetate |
| Ethinyl estradiol; Levonorgestrel |
| Ethinyl estradiol; Norethindrone |
| Ethinyl estradiol; Norethindrone |
| acetate |
| Ethinyl estradiol; Norgestimate |
| Ethinyl estradiol; Norgestrel |
| Etidronate disodium |
| Etodolac |
| Etoposide |
| etoricoxib |
| exendin-4 |
| famciclovir |
| |
| Famotidine |
| Felodipine |
| fenofibrate |
| fenretinide |
| Fentanyl |
| |
| fexofenadine hydrochloride |
| filgrastim SD01 |
| finasteride |
| flecainide acetate |
| fluconazole |
| |
| Fludrocortisone acetate |
| flumazenil |
| Fluoxetine |
| Flutamide |
| fluvastatin |
| Fluvoxamine maleate |
| |
| follitropin alfa/beta |
| Formoterol |
| Fosinopril |
| fosphenytoin sodium |
| Furosemide |
| Gabapentin |
| gadodiamide |
| gadopentetate dimeglumine |
| gadoteridol |
| ganaxolone |
| ganciclovir |
| gantofiban |
| gastrin CW17 immunogen |
| gemcitabine hydrochloride |
| |
| Gemfibrozil |
| |
| Gentamicin Isoton |
| gepirone hydrochloride |
| glatiramer acetate |
| |
| glimepiride |
| Glipizide |
| Glucagon HCL |
| Glyburide |
| granisetron hydrochloride |
| |
| Haloperidal |
| BMS 284756 |
| Hydrochlorothiazid |
| Hydrochlorothiazide; Triamterene |
| Hydromorphone HCL |
| Hydroxychloroquine sulfate |
| Ibuprofen |
| Idarubicin HCL |
| ilodecakin |
| ilomastat |
| imiglucerase |
| Imipramine HCL |
| indinavir sulfate |
| infliximab |
| insulin lispro |
| interferon alfacon-1 |
| interferon beta-1a |
| |
| interleukin-2 |
| iodixanol |
| iopromide |
| loxaglate meglumine; loxaglate |
| sodium |
| Ipratropium |
| Irbesartan |
| irinotecan hydrochloride |
| Isosorbide Dinitrate |
| Isotretinoin |
| Isradipine |
| itasetron |
| itraconazole |
| Ketoconazole |
| Ketoprofen |
| Ketorolac |
| Ketotifen |
| Labetalol HCL |
| lamivudine |
| lamivudine; zidovudine |
| lamotrigine |
| lansoprazole |
| lansoprazole, amoxicillin, |
| clarithromycin |
| leflunomide |
| lesopitron |
| Leuprolide acetate |
| levocarnitine |
| levocetirizine |
| Levofloxacin |
| Levothyroxine |
| lintuzumab |
| Lisinopril |
| lisinopril; hydrochlorothiazide |
| CS 834 |
| Loperamide HCL |
| Loracarbef |
| loratadine |
| Lorazepam |
| losartan potassium |
| |
| losartan potassium; |
| hydrochlorothiazide |
| Lovastatin |
| marimastat |
| mecasermin |
| Medroxyprogesterone Acetate |
| mefloquine hydrochloride |
| megestrol acetate |
| CVT CW124 |
| Mercaptopurine |
| Meropenem |
| mesalamine |
| |
| mesna |
| Metaxalone |
| Metfomin |
| EM 800 |
| Methylphenidate HCL |
| Methylprednisolone Acetate |
| FK 463 |
| Metolazone |
| metoprolol succinate |
| MK 826 |
| Metronidazole |
| milrinone lactate |
| Minocycline HCL |
| mirtazapine |
| Misoprostol |
| mitiglinide |
| mitoxantrone hydrochloride |
| mivacurium chloride |
| modafinil |
| moexepril hydrochloride |
| montelukast sodium |
| |
| Morphine Sulfate |
| Mycophenolate mofetil |
| nabumetone |
| |
| Nadolol |
| Naproxen sodium |
| naratriptan hydrochloride |
| nefazodone hydrochloride |
| nelarabine |
| nelfinavir mesylate |
| |
| nesiritide |
| nevirapine |
| nifedipine |
| nimodipine |
| nisoldipine |
| nitrofurantoin, nitrofurantoin, |
| macrocrystalline |
| Nitroglycerin |
| nizatidine |
| |
| norastemizole |
| Norethindrone |
| norfloxacin |
| Nortriptyline HCL |
| octreotide acetate |
| Oxycodone/APAP |
| ofloxacin |
| olanzapine |
| Omeprazole |
| ondansetron hydrochloride |
| oprelvekin |
| orlistat |
| Orphenadrine citrate |
| Oxaprozin |
| Oxazepam |
| oxybutynin chloride |
| Oxycodone HCL |
| GM 611 |
| M-CSF |
| pagoclone |
| palivizumab |
| pamidronate disodium |
| paricalcitrol |
| paroxetine hydrochloride |
| pemetrexed |
| Pemoline |
| penicillin V |
| pentosan polysulfate sodium |
| Pentoxifylline |
| Pergolide |
| NE 0080 |
| Phenobarbital |
| Phenytoin sodium |
| ploglitazone hydrochloride |
| Piperacillin sodium |
| pleconaril |
| poloxamer CW188 |
| posaconazole |
| NN 304 |
| pramipexole dihydrochloride |
| pravastatin sodium |
| Prednisone |
| pregabalin |
| Primidone |
| prinomastat |
| Prochlorperazine maleate |
| Promethazine HCL |
| PD 135158 |
| Propoxyphene-N/APAP |
| Propranolol HCL |
| prourokinase |
| quetiapine fumarate |
| quinapril hydrochloride |
| rabeprazole sodium |
| raloxifine hydrochloride |
| Ramipril |
| Ranitidine |
| ranolazine hydrochloride |
| relaxin |
| remacemide |
| repaglinide |
| repinotan |
| ribavirin+peginterferon alfa-2b |
| riluzole |
| Rimantadine HCL |
| risperidone |
| ritonavir |
| rizatriptan benxoate |
| rocuronium bromide |
| rofecoxib |
| ropinirole hydrochloride |
| rosiglitazone maleate |
| Goserelin |
| rubitecan |
| sagramostim |
| saquinavir |
| Docetaxel |
| satraplatin |
| Selegiline HCL |
| sertraline hydrochloride |
| sevelamer hydrochloride |
| sevirumab |
| sibutramine hydrochloride |
| sildenafil citrate |
| simvastatin |
| sinapultide |
| sitafloxacin |
| sodium polystyrene sulfonate |
| Sotalol HCL |
| sparfosic acid |
| Spironolactone |
| stavudine |
| sucralfate |
| sumatriptan |
| tabimorelin |
| tamoxifen citrate |
| tamsulosin hydrochloride |
| Temazepam |
| tenofovir disoproxil |
| tepoxalin |
| Terazosin HCL |
| terbinafine hydrochloride |
| terbutaline sulfate |
| teriparatide |
| tetracycline |
| thalidomide |
| Theophylline |
| Thiotepa |
| thrombopoetin, TPO |
| tiagabine hydrochloride |
| ticlopidine hydrochloride |
| tifacogin |
| tirapazamine |
| tirofiban hydrochloride |
| tizanidine hydrochloride |
| Tobramycin sulfate |
| tolterodine tartrate |
| tomoxetine |
| topiramate |
| Topotecan HCL |
| toresemide |
| tPA analogue |
| Tramadol HCL |
| trandolapril |
| trastuzumab |
| Trazadone HCL |
| Triamterene/HCTZ |
| troglitazone |
| trovafloxacin mesylate |
| urokinase |
| Ursodiol |
| valacyclovir hydrochloride |
| valdecoxib |
| Valproic Acid |
| valsartan, hydrochlorothiazide |
| valspodar |
| Vancomycin HCL |
| Vecuronium bromide |
| venlafaxine hydrochloride |
| Verapamil HCL |
| vinorelbine tartrate |
| Vitamin B12 |
| Vitamin C |
| voriconazole |
| Warfarin Sodium |
| xaliproden |
| zafirlukast |
| zaleplon |
| zenarestat |
| zidovudine |
| zolmitriptan |
| Zolpidem |
| bleomycin |
| Phytoseterol |
| paclitazel |
| Flutiasone |
| Fluorouracil |
| Pseudoephedrine |
| A 78773 |
| AGI 1067 |
| BCX CW1812 |
| BMS CW188667 |
| BMS CW193884 |
| BMS CW204352 |
| BPI 21 |
| CD11a |
| CEB 925 |
| Propofol |
| GT 102279 |
| Recombinant hepatitis vaccine |
| L 159282 |
| LFA3TIP |
| Daily Multi Vit |
| Erythromycn/Sulfsx |
| Ethinyl estradiol; Desogestrel |
| Lithium Carbonate |
| LYM 1 |
| Methylprednisolone Sodium |
| succinate |
| rotavirus vaccine |
| saquinavir mesylate |
| arginine |
| heparin |
| Thymosin alpha |
| montelukast sodium and |
| fexofenadine hydrochloride |
| lodothyronine |
| lodothyronine and thyroxine |
| Codeine |
| Ethylmorphine |
| Diacetylmorphine |
| Hydromorphone |
| Hydrocodone |
| Oxymorphone |
| Dihydrocodeine |
| Dihydromorphine |
| Methyldihydromorphinone |
| Codeine and promethazine |
| Codeine, phenylephrine and |
| promethazine |
| Codeine and guaifenesin |
| Codeine, guaifenesin and |
| pseudoephedrine |
| Aspirin, carisoprodol and codeine |
| Himatropine methylbromide and hydrocodone bitartrate |
| Hydrocodone bitartrate and phenylpropanolamine |
| Acetaminophen and hydrocodone bitartrate |
| Chlorpheniramine maleate, Hydrocodone bitartrate and pseudoephedrine |
| Guaifenesin and hydrocodone Ibuprofen and hydrocodone |
| Chlorpheniramine polistirex and hydrocodone polystirex |
| naltrexone |

Preferably, the polypeptide is (i) an oligopeptide, (ii) a homopolymer of one of the twenty naturally occurring amino acids (L or D isomers), or an isomer, analogue, or derivative thereof, (iii) a heteropolymer of two or more naturally occurring amino acids (L or D isomers), or an isomer, analogue, or derivative thereof, (iv) a homopolymer of a synthetic amino acid, (v) a heteropolymer of two or more synthetic amino acids or (vi) a heteropolymer of one or more naturally occurring amino acids and one or more synthetic amino acids.

Proteins, oligopeptides and polypeptides are polymers of amino acids that have primary, secondary and tertiary structures. The secondary structure of the protein is the local conformation of the polypeptide chain and consists of helices, pleated sheets and turns. The protein's amino acid sequence and the structural constraints on the conformations of the chain determine the spatial arrangement of the molecule. The folding of the secondary structure and the spatial arrangement of the side chains constitute the tertiary structure.

Proteins fold because of the dynamics associated between neighboring atoms on the protein and solvent molecules. The thermodynamics of protein folding and unfolding are defined by the free energy of a particular condition of the protein that relies on a particular model. The process of protein folding involves, amongst other things, amino acid residues packing into a hydrophobic core. The amino acid side chains inside the protein core occupy the same volume as they do in amino acid crystals. The folded protein interior is therefore more like a crystalline solid than an oil drop and so the best model for determining forces contributing to protein stability is the solid reference state.

The major forces contributing to the thermodynamics of protein folding are Van der Waals interactions, hydrogen bonds, electrostatic interactions, configurational entropy and the hydrophobic effect. Considering protein stability, the hydrophobic effect refers to the energetic consequences of removing apolar groups from the protein interior and exposing them to water. Comparing the energy of amino acid hydrolysis with protein unfolding in the solid reference state, the hydrophobic effect is the dominant force. Hydrogen bonds are established during the protein fold process and intramolecular bonds are formed at the expense of hydrogen bonds with water. Water molecules are "pushed out" of the packed, hydrophobic protein core. All of these forces combine and contribute to the overall stability of the folded protein where the degree to which ideal packing occurs determines the degree of relative stability of the protein. The result of maximum packing is to produce a center of residues or hydrophobic core that has maximum shielding from solvent.

Since it is likely that lipophilic drugs would reside in the hydrophobic core of a peptide, it would require energy to unfold the peptide before the drug can be released. The unfolding process requires overcoming the hydrophobic effect by hydrating the amino acids or achieving the melting temperature of the protein. The heat of hydration is a destabilization of a protein. Typically, the folded state of a protein is favored by only 5-15 kcal/mole over the unfolded state. Nonetheless, protein unfolding at neural pH and at room temperature requires chemical reagents. In fact, partial unfolding of a protein is often observed prior to the onset of irreversible chemical or conformation processes. Moreover, protein conformation generally controls the rate and extent of deleterious chemical reactions.

Conformational protection of active agents by proteins depends on the stability of the protein's folded state and the thermodynamics associated with the agent's decomposition. Conditions necessary for the agent's decomposition should be different than for protein unfolding.

Selection of the amino acids will depend on the physical properties desired. For instance, if increase in bulk or lipophilicity is desired, then the carrier polypeptide will be enriched in the amino acids in the table provided below. Polar amino acids, on the other hand, can be selected to increase the hydrophilicity of the polypeptide.

Ionizing amino acids can be selected for pH controlled peptide unfolding. Aspartic acid, glutamic acid and tyrosine carry a neutral charge in the stomach, but will ionize upon entry into the intestine. Conversely, basic amino acids, such as histidine, lysine and arginine, ionize in the stomach and are neutral in an alkaline environment.

Other factors such as π-π interactions between aromatic residues, kinking of the peptide chain by addition of proline, disulfide crosslinking and hydrogen bonding can all be used to select the optimum amino acid sequence for a given application. Ordering of the linear sequence can influence how these interactions can be maximized and is important in directing the secondary and tertiary structures of the polypeptide.

Furthermore, amino acids with reactive side chains (e.g., glutamic acid, lysine, aspartic acid, serine, threonine and cysteine) can be incorporated for attaching multiple active agents or adjuvants to the same carrier peptide. This is particularly useful if a synergistic effect between two or more active agents is desired.

As stated above, variable molecular weights of the carrier compound can have profound effects on the active agent release kinetics. As a result, low molecular weight active agent delivery systems are preferred. An advantage of this invention is that chain length and molecular weight of the polypeptide can be optimized depending on the level of conformational protection desired. This property can be optimized in concert with the kinetics of the first order release mechanism. Thus, another advantage of this invention is that prolonged release time can be imparted by increasing the molecular weight of the carrier polypeptide. Another, significant advantage of the invention is that the kinetics of active agent release is primarily controlled by the enzymatic hydrolysis of the key bond between the carrier peptide and the active agent.

Dextran is the only polysaccharide known that has been explored as a macromolecular carrier for the covalent binding of drug for colon specific drug delivery. Generally, it was only possible to load up to 1/10 of the total drug-dextran conjugate weight with drug. As stated earlier, polysaccharides are digested mainly in the colon and drug absorption is mainly limited to the colon. As compared to dextran, this invention has two major advantages. First, peptides are hydrolyzed by any one of several aminopeptidases found in the intestinal lumen or associated with the brush-border membrane and so active agent release and subsequent absorption can occur in the jejunum or the ileum. Second, the molecular weight of the carrier molecule can be controlled and, thus, active agent loading can also be controlled.

As a practical example, the following table lists the molecular weights of lipophilic amino acids (less one water molecule) and selected analgesics and vitamins.

**TABLE 2**

| Amino acid | MW | Active agent | MW |
|---|---|---|---|
| | | | |
| Glycine | 57 | Acetaminophen | 151 |
| Alanine | 71 | Vitamin B₆ (Pyroxidine) | 169 |
| Valine | 99 | Vitamin C (Ascorbic acid) | 176 |
| Leucine | 113 | Aspirin | 180 |
| Isoleucine | 113 | Ibuprofen | 206 |
| Phenylalanine | 147 | Retinoic acid | 300 |
| Tyrosine | 163 | Vitamin B₂ (Riboflavin) | 376 |
| | | Vitamin D₂ | 397 |
| | | Vitamin E (Tocopherol) | 431 |

Lipophilic amino acids are preferred because conformational protection through the stomach is important for the selected active agents, which were selected based on ease of covalent attachment to an oligopeptide. Eighteen was subtracted from the amino acid's molecular weight so that their condensation into a polypeptide is considered. For example, a decamer of glycine (MW=588) linked to aspirin would have a total molecular weight of 750 and aspirin would represent 24% of the total weight of the active agent delivery composition or over two times the maximum drug loading for dextran. This is only for an N- or C- terminus application, for those active agents attached to pendant groups of decaglutamic acid, for instance, a drug with a molecular weight of 180 could conceivably have a loading of 58%, although this may not be entirely practical.

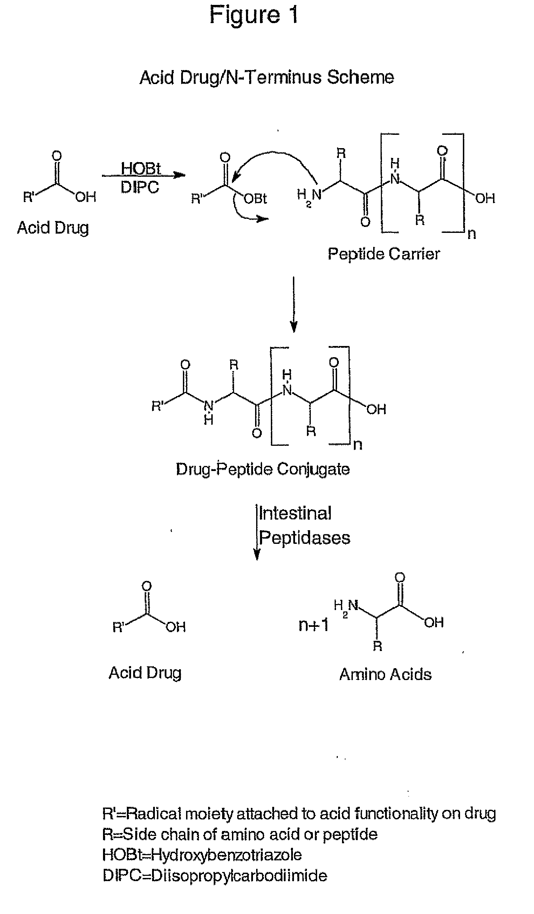

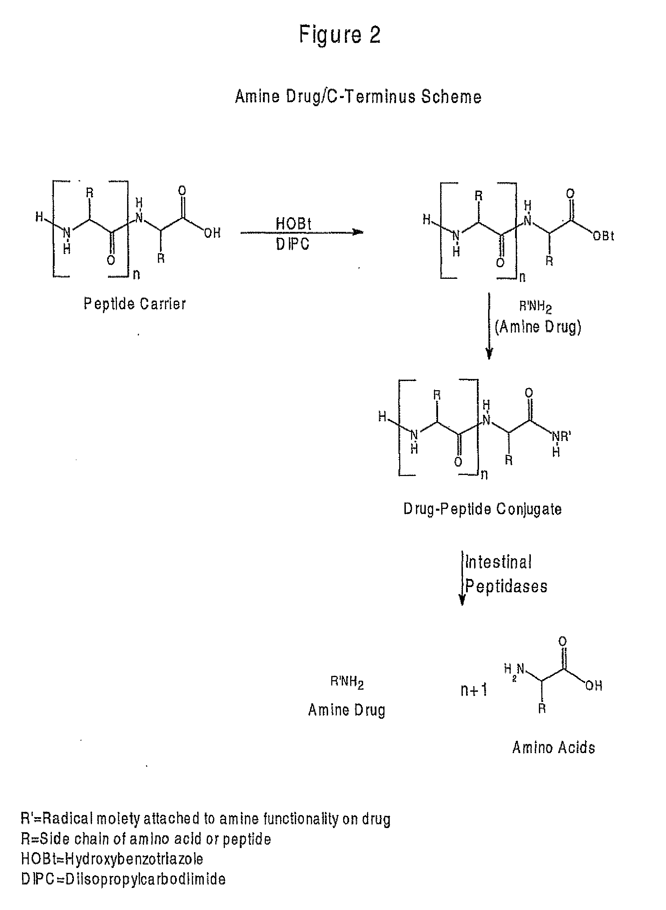

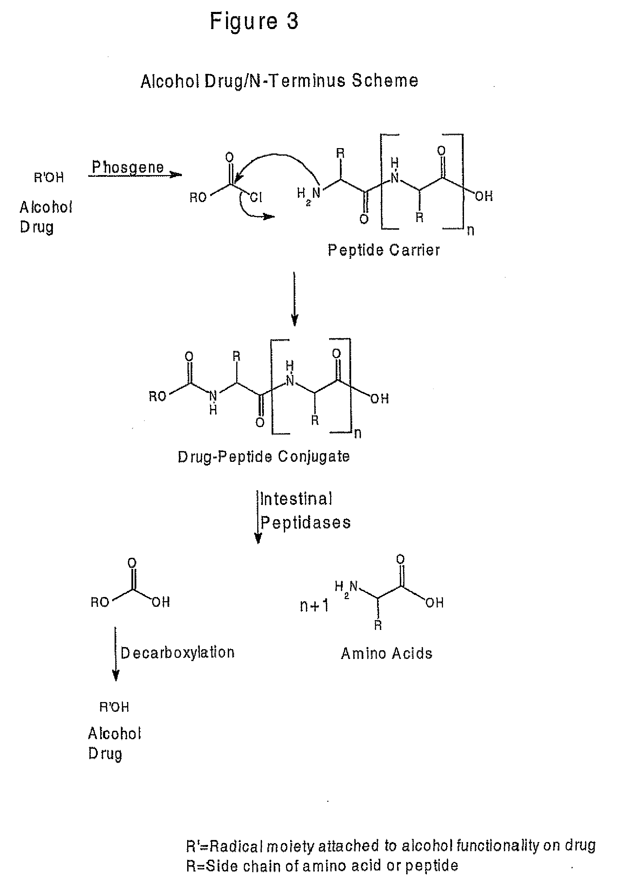

The alcohol, amine or carboxylic acid group of the active agent is covalently attached to the N-terminus, the C-terminus or the side chain of the oligopeptide or polypeptide. The location of attachment depends somewhat on the functional group selection. For instance, if the active drug is a carboxylic acid (e.g., aspirin) then the N-terminus of the oligopeptide is the preferred point of attachment as shown in Fig. 1. If the active agent is an amine (e.g., ampicillin), then the C-terminus is the preferred point of attachment in order to achieve a stable peptide linked active agent as shown in Fig. 2. In both, the C- and N-terminus examples, the peptide is, in essence, extended by one monomeric unit forming a new peptide bond. If the active agent is an alcohol, then either the C-terminus or the N-terminus is the preferred point of attachment in order to achieve a stable composition. As in the example above where the alcohol, norethindrone, was covalently attached to poly(hydroxypropylglutamine), an alcohol can be converted into an alkylchloroformate with phosgene. This invention, then, pertains to the reaction of this key intermediate with the N-terminus of the peptide carrier as shown in Fig. 3. Figs. 1 through 3 also depict the release of the active ingredient from the peptide carrier by intestinal peptidases.

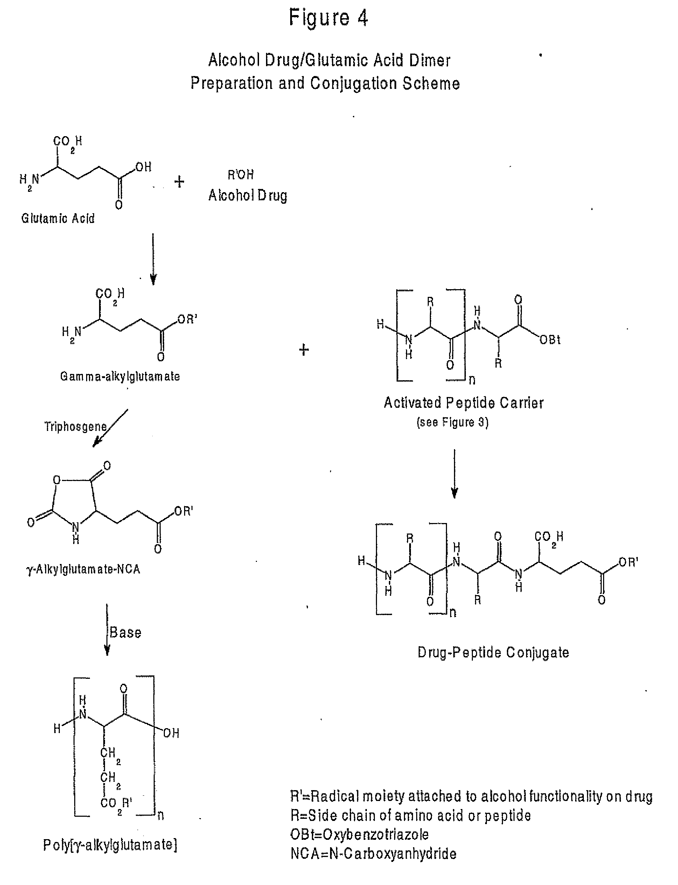

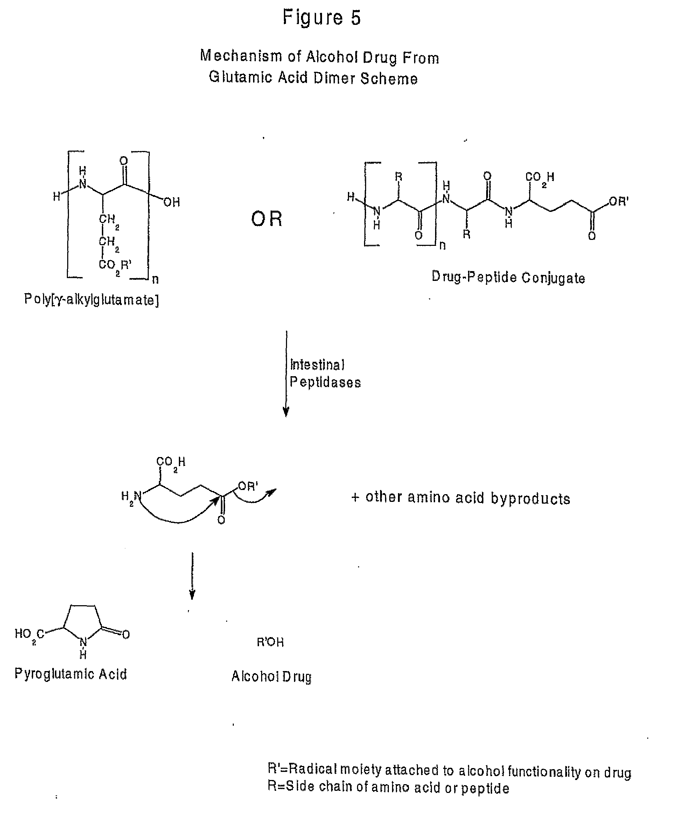

The alcohol can be selectively bound to the gamma carboxylate of glutamic acid and then this conjugate covalently attached to the C-terminus of the peptide carrier. Because the glutamic acid-drug conjugate can be considered a dimer, this product adds two monomeric units to the C-terminus of the peptide carrier where the glutamic acid moiety serves as a spacer between the peptide and the drug as shown in Fig. 4. Intestinal enzymatic hydrolysis of the key peptide bond releases the glutamic acid-drug moiety from the peptide carrier. The newly formed free amine of the glutamic acid residue will then undergo an intramolecular transamination reaction, thereby, releasing the active agent with coincident formation of pyroglutamic acid as shown in Fig. 5. Alternatively, the glutamic acid-drug dimer can be converted into the gamma ester of glutamic acid N-carboxyanhydride. This intermediate can then be polymerized, as described above, using any suitable initiator as shown in Fig. 4. The product of this polymerization is polyglutamic acid with active ingredients attached to multiple pendant groups. Hence, maximum drug loading of the carrier peptide can be achieved. In addition, other amino acid-NCA's can be copolymerized with the gamma ester glutamic acid NCA to impart specific properties to the drug delivery system.

The invention also provides a method of imparting the same mechanism of action for other polypeptides containing functional side chains. Examples include, but are not limited to, polylysine, polyasparagine, polyarginine, polyserine, polycysteine, polytyrosine, polythreonine and polyglutamine. The mechanism can translate to these polypeptides through a spacer or linker on the pendant group, which is terminated, preferably, by the glutamic acid-drug dimer. This carrier peptide-drug conjugate is distinguished from the prior art by virtue of the fact that the primary release of the drug moiety relies on peptidases and not on esterases. Alternatively, the active agent can be attached directly to the pendant group where some other indigenous enzymes in the alimentary tract can affect release.

The active agent can be covalently attached to the N-terminus, the C-terminus or the side chain of the polypeptide using known techniques. Examples of linking organic compounds to the N-terminus type of a peptide include, but are not limited to, the attachment of naphthylacetic acid to LH-RH, coumarinic acid to opioid peptides and 1,3-dialkyl-3-acyltriazenes to tetragastrin and pentagastrin. As another example, there are known techniques for forming peptide linked biotin and peptide linked acridine.

The polypeptide carrier can be prepared using conventional techniques. A preferred technique is copolymerization of mixtures of amino acid N-carboxyanhydrides. Alternatively, if a specific sequence is desired, a solid state automated peptide synthesizer can be used.

The addition of stabilizers to the composition has the potential of stabilizing the polypeptide further. Stabilizers such as sugar, amino acids, polyethylene glycol (PEG) and salts have been shown to prevent protein unfolding. In another embodiment of the invention, a pre-first order release of the active agent is imparted by microencapsulating the carrier polypeptide-active agent conjugate in a polysaccharide, amino acid complex, PEG or salts.

There is evidence that hydrophilic compounds are absorbed through the intestinal epithelia efficiently via specialized transporters. The entire membrane transport system is intrinsically asymmetric and responds asymmetrically to cofactors. Thus, one can expect that excitation of the membrane transport system will involve some sort of specialized adjuvant resulting in localized delivery of active agents. There are seven known intestinal transport systems classified according to the physical properties of the transported substrate. They include the amino acid, oligopeptide, glucose, monocarboxic acid, phosphate, bile acid and the P-glycoprotein transport systems and each has its own associated mechanism of transport. The mechanisms can depend on hydrogen ions, sodium ions, binding sites or other cofactors. The invention also allows targeting the mechanisms for intestinal epithelial transport systems to facilitate absorption of active agents.

In another embodiment of the invention, the composition includes one or more adjuvants to enhance the bioavailability of the active agent. Addition of an adjuvant is particularly preferred when using an otherwise poorly absorbed active agent. Suitable adjuvants, for example, include: papain, which is a potent enzyme for releasing the catalytic domain of aminopeptidase-N into the lumen; glycorecognizers, which activate enzymes in the BBM; and bile acids, which have been attached to peptides to enhance absorption of the peptides.

Preferably, the resultant peptide-active agent conjugate is formulated into a tablet using suitable excipients and can either be wet granulated or dry compressed.

Compositions of the invention are, in essence, the formation of amides from acids and amines and can be prepared by the following examples.

### Acid/N-terminus conjugation (Fig. 1)

An acid bioactive agent can be dissolved in DMF under nitrogen and cooled to 0 °C. The solution can then be treated with diisopropylcarbodiimide and hydroxybenzotriazole followed by the amine peptide carrier. The reaction can then be stirred for several hours at room temperature, the urea by-product filtered off, the product precipitated out in ether and purified using gel permeation chromatography (GPC) or dialysis.

### Amine/C-terminus conjugation (Fig. 2)

The peptide carrier can be dissolved in DMF under nitrogen and cooled to 0 °C. The solution can then be treated with diisopropylcarbodiimide and hydroxybenzotriazole followed by the amine bioactive agent. The reaction can then be stirred for several hours at room temperature, the urea by-product filtered off, the product precipitated out in ether and purified using GPC or dialysis.

### Alcohol/N-Terminus Conjugation (Fig. 3)

In the following example the combination of the alcohol with triphosgene produces a chloroformate, which when reacted with the N-terminus of the peptide produces a carbamate. Pursuant to this, an alcohol bioactive agent can be treated with triphosgene in dry DMF under nitrogen. The suitably protected peptide carrier is then added slowly and the solution stirred at room temperature for several hours. The product is then precipitated out in ether The crude product is suitably deprotected and purified using GPC.

Other solvents, activating agents, cocatalysts and bases can be used. Examples of other solvents include dimethylsulfoxide, ethers such as tetrahydrofuran or chlorinated solvents such as chloroform. Examples of other activating agents include dicyclohexylcarbodiimide or thionyl chloride. An example of another cocatalyst is N-hydroxysuccinimide. Examples of bases include pyrrolidinopyridine, dimethylaminopyridine, triethylamine or tributylamine.

### Preparation of γ-Alkyl Glutamate (Fig. 4)

There have been over 30 different γ-alkyl glutamates prepared any one of which may be suitable for the drug alcohol of choice. For example, a suspension of glutamic acid, the alcohol and concentrated hydrochloric acid can be prepared and heated for several hours. The γ-alkyl glutamate product can be precipitated out in acetone, filtered, dried and recrystallized from hot water.

### γ-Alkyl Glutamate/C-Terminus Conjugation (Fig. 4)

The peptide carrier can be dissolved in DMF under nitrogen and cooled to 0 °C. The solution can then be treated with diisopropylcarbodiimide and hydroxybenzotriazole followed by the γ-alkyl glutamate bioactive agent. The reaction can then be stirred for several hours at room temperature, the urea by-product filtered off, the product precipitated out in ether and purified using GPC or dialysis.

### Preparation of γ-Alkyl Glutamate-NCA

γ-Alkyl glutamate can be suspended in dry THF where triphosgene is added and the mixture refluxed under a nitrogen atmosphere until the mixture becomes homogenous. The solution can be poured into heptane to precipitate the NCA product, which is filtered, dried and recrystallized from a suitable solvent.

### Preparation of Poly[γ-Alkyl Glutamate]

γ-Alkyl glutamate-NCA can be dissolved in dry DMF where a catalytic amount of a primary amine can be added to the solution until it becomes viscous (typically overnight). The product can be isolated from the solution by pouring it into water and filtering. The product can be purified using GPC or dialysis.

### EXAMPLE 1

### Preparation of Capped Iodothyronine Composition Comprising a Copolymer of T₃ and T₄ Covalently Attached to the N-terminus of Polyglutamic Acid

The synthesis of polyglutamic acid is well known through a variety of reported methods. For the present examples polyglutamic acid was synthesized through the activation of the Benzyl Glutamic NCA (BnGlu-NCA) monomer. The BnGlu-NCA was then polymerized and the benzyl groups removed with hydrogen bromide. When capping polyglutamic acid, the liberation of its N-terminus amino group from the hydrogen bromide complex without imparting unwanted nucleophilicity to the free carboxylic acids is critical. Reactions using sodium carbonate, sodium bicarbonate, and sodium acetate produced glutamic acid/T₄/T₃ copolymer with the T₄ and T₃ incorporation decreasing with increasing pKb. Sodium acetate was the preferred reagent because its pKb is between that of sodium bromide, polyglutamic acid, and sodium salt. The reaction using basic alumina kept the T₄-NCA and T₃-NCA intact with no apparent capping or self-polymerization. The stability of T₄-NCA and T₃-NCA will influence how .glutamic acid/T₄/T₃ copolymer will be commercially manufactured. Sodium acetate can be replaced with sodium carbonate, sodium bicarbonate, sodium propionate, sodium butyrate, sodium pivalate, basic alumina, or any other weak base capable of neutralizing hydrogen bromide complexed with an amino group.

The synthesis of glutamic acid/T₄/T₃ copolymer began with benzylglutamic acid, thyroxine, and triiodothyronine. Each of these synthons was independently reacted with triphosgene in a suitable organic solvent. The BnGlu-NCA was polymerized in tetrahydrofuran (THF) with sodium methoxide as an initiator. Polybenzylglutamic acid was deprotected with 15% hydrogen bromide in acetic acid. This product needs to be free of uncomplexed hydrogen bromide where it was dissolved in DMF and treated with sodium acetate. The previously prepared T₄-NCA and T₃-NCA were blended and added to the solution. The reaction was then stirred until T₄-NCA or T₃-NCA were no longer detected by thin layer chromatography (TLC). The final product was added to water and the precipitate was washed with water and dried *in vacuo* to yield a white amorphous powder.

Experimentation with several weak bases revealed that a variety of sodium salts of a carboxylic acid work in capping polyglutamic acid. The reaction was tried with sodium propionate, sodium butyrate, and sodium pivalate in lieu of sodium acetate all with essentially the same result.

### Preparation of benzylglutamic acid-NCA

Benzylglutamic acid (25 grams) was suspended in 400 mL anhydrous ethyl acetate under nitrogen. The mixture was heated to reflux where 30 grams of triphosgene was added in six (6) equal portions. The reaction was refluxed for three (3) hours until homogenous. The solution was cooled to room temperature, filtered, and concentrated *in vacuo*. The white powder was recrystallized from 50 mL of hot anhydrous ethyl acetate to yield 17.4 grams (63%) of a white powder.

### Preparation of T₄-NCA

In a round bottom flask fitted with a nitrogen inlet, five grams of thyroxine was stirred with 25 mL of tetrahydrofuran (THF) and 1.3 grams of triphosgene and the mixture refluxed for four (4) hours until homogenous. The solution was cooled to room temperature, and added dropwise to 200 mL of heptane with stirring. The crystals were filtered and dried *in vacuo* to yield 4.72 grams (91%) of an off-white powder.

### Preparation of T₃-NCA

In a round bottom flask fitted with a nitrogen inlet, 4.29 grams of triiodothyronine was stirred with 20 mL of tetrahydrofuran (THF) and 1.45 grams of triphosgene and the mixture refluxed for four (4) hours until homogenous. The solution was cooled to room temperature and added dropwise to 200 ml of heptane with stirring. The liquid was decanted off the yellow gum, which was recrystallized, from anhydrous ethyl acetate and hexanes to yield 2.5 grams (56%) of a white powder that was dried under high vacuum.

### Preparation of polybenzylglutamic acid

Benzylglutamic acid (17.4 grams) was dissolved in anhydrous tetrahydrofuran (THF) under nitrogen where 238 mg of sodium methoxide was added portionwise. The solution was stirred for two (2) days with a marked increase in viscosity. The solution was poured into 1.5 L of petroleum ether with stirring. The petroleum ether was decanted off and an additional 1L of petroleum ether was added back. The mixture was stirred by hand, the petroleum ether was decanted off and the process repeated with 500 mL of petroleum ether. The white solid was air dried and then vacuum dried to yield 14.7 (95%) of a white fluffy paper-like solid.

### Preparation of polyglutamic acid

Acetic acid (10mL) was stirred with 0 mL 30wt% hydrogen bromide (HBr) in acetic acid where 1.96 of polybenzylglutamic acid was added by hand. The mixture was stirred at room temperature for one day and was, then, added to 50 mL of ether. The white precipitant was filtered, washed with 4 x 30 mL of ether and dried under a high vacuum to yield 1.11 grams (97%) of a white powder.

### Preparation of glutamic acid/T₄/T₃ copolymer

Polyglutamic acid (375 mg) was dissolved in dry 3 mL DMF. Sodium acetate (24 mg) was added followed by a blend of 105 mg of T₄-NCA and 8 mg of T₃-NCA. The solution was stirred for two (2) days where TLC showed the absence of thyronine starting materials. The solution was poured into 30 mL of water and cooled 10 °C overnight. The precipitant was filtered, washed with water, and dried under high vacuum to yield 413 mg (85%) of light beige powder. The proton NMR revealed a copolymer of T₃ and T₄ covalently attached to the N-terminus of polyglutamic acid, which was virtually completely digested by the pronase enzyme system in two hours.

### EXAMPLE 2

### Preparation of Peptide Polymers

**Polyaspartic acid:** Asp(OtBu) (13mg, 0.07mmol) and Asp(OtBu)-NCA (200mg, 0.93 mmol) were dissolved in anhydrous DMF (5ml), and the solution allowed to stir over night at room temperature under argon. The following morning, 2.5 ml of the reaction mixture was transferred to separate flask (Flask B). T4-NCA (27mg, 0.03mmol) was added to the original flask (Flask A), and both solutions were allowed to continue stirring under argon for an additional 24 hours. Polymer was then precipitated by the addition of water (50ml) to each flask. The resulting solids were collected by filtration and dried over night under vacuum.

The dried Asp(OtBu)ₙ (Flask B) and T4-Asp(OtBu)ₙ (Flask A) were then dissolved in 95% trifluoroacetic acid in water (3ml) and allowed to stir at room temperature for 2 hours. The deprotected polymers were then precipitated by the addition of ethyl ether (10ml) and then storing the suspension at 4 °C for 2 hours. The respective polymers were then collected by filtration and the solids dried over night under vacuum. This afforded 48mg of Aspₙ (Flask B) and 12mg of T4-Aspₙ (Flask A). MALDI indicated that T4-Aspₙ (Flask A) consisted of a mixture of polymers of varying lengths: T4-Asp₃₋₁₂.

**Polyserine** and **Polythreonine** were also prepared using this protocol. The serine reaction mixture contained N-methylmorpholine (1.1 equivalents).

| Amino acid derivative | Polymer | Isolated | Percent yield | Mass Range |
|---|---|---|---|---|
| Asp(OtBu) | Asp(OtBu)ₙ | 48mg | 84% | NA |
| | T4-Asp(OtBu)ₙ | 12mg | 14% | T4-Asp₃₋₁₂ |
| Ser(OtBu) | Ser(OtBu)ₙ | 73mg | 101%³ | Ser₇₋₈ |
| | T4-Ser(OtBu)ₙ | 50mg | 43% | T4-Ser₄₋₉ |
| Thr(OtBu) | Thr(OtBu)ₙ | 29mg | 20% | Thr₇₋₈ |
| | T4-Thr(OtBu)ₙ | 66mg | 24% | T4-Thr₁₋₈ |

The percent yield was estimated based on the total amino acid content in the original reaction prior to splitting the reaction. The Mass range was determined from MALDI. The yield over 100% could reflect either the presence of salts or uneven distribution when the reaction mixture was split.

HPLC and Pronase experiments indicate little to no free T4 is present in the T4-Asp₃₋₁₂, T4-Ser₄₋₉ and T4-Thr₁₋₈ samples, and that T4 is liberated upon digestion.

### N-carboxyanhydrides

N-carboxyanhydrides (NCA's) of the amino acids listed below were prepared using a protocol similar ) to that reported for glutamic acid. Minor variations in their final workups are noted below.

| **Amino Acid** | **Chemical Shift in the NCA** | | | |
|---|---|---|---|---|
| | **α** | **β** | **γ** | **other (OtBu)** |
| Alanine | 4.41 (q, 1H) | 1.57 (d, 3H) | | |
| | | | | |
| Valine | 4.20 (d, 1H) | 2.28-2.19 (m, 1H) | 1.08 (d, 3H) | |
| | | | 1.02 (d, 3H) | |
| Serine (OtBu) | 4.58 (m, 1H) | 3.62 (dd, 1H) | | 1.10 (s, 9H) |
| | | 3.50 (dd, 1H) | | |
| | | | | |
| Aspartic acid (OtBu) | 4.51 (dd,1H) | 2.93 (dd, 1H) | | 1.44 (s, 9H) |
| | | 2.73 (dd, 1H) | | |
| | | | | |
| Glutamic acid (OtBu) | 4.34 (dd,1H) | 2.28-2.20 (m, 1H) | 2.45 (t, 2H) | 1.44 (s, 9H) |
| | | 2.09-1.99(m, 1H) | | |

| **Amino Acid** | **Isolation of NCA** | | | |
|---|---|---|---|---|
| Alanine | precipitate with hexanes in 68% yield | | | |
| | | | | |
| Valine | precipitate with hexanes in 89% yield | | | |
| | | | | |
| Serine (OtBu) | suspended in isopropanol and precipitated with hexanes in 83% yield | | | |
| | | | | |
| Aspartic acod (OtBu) | suspended in isopropanol and precipitated with hexanes in 55% yield | | | |
| | | | | |
| Glutamic acid (OtBu) | suspended in isopropanol and precipitated with hexanes in 77% yield | | | |

### EXAMPLE 3

### Preparation of (Glu)ₙ-Cephalexin

Glu(OtBu)NCA (1.000 g, 4.4mmol) and Cephalexin•HCl (0.106g, 0.3mmol) were dissolved in anhydrous DMF (5ml). The reaction was then allowed to stir at room temperature under argon. After 3 days, the solvent was removed by rotary-evaporation under vacuum. The resulting solid was then placed under argon and then dissolved in 4N HCl in Dioxane (2ml) and then allowed to stir at room temperature under a blanket of argon. After 1 hour, the dioxane and HCl were removed by rotary-evaporation under vacuum. The solid was then suspended in methanol (2ml) and once more brought to dryness by rotary-evaporation in order to remove residual HCl and dioxane. This material was then resuspended in methanol (2ml) and precipitated by the addition of water (20ml). The aqueous suspension was then stored at 4°C for 4 hours, and the solid isolated by centrifugation. The pelleted material was then allowed to dry under vacuum over night. This process afforded a mixture of (Glu)ₙ and (Glu)ₙ-cephalexin (464mg) as determined by MALDI. MALDI indicates a mixture of polymers (Glu)₇₋₁₃ and (Glu)₅₋₁₄-cephalexin. Other chain-lengths may be present but they are not clearly visible in the MALDI spectra. Reversed-phase HPLC (265nm detection, C18 column, 16%MeOH/4%THF/80%water mobile phase) indicated that no free cephalexin was present in the isolated material. "Water" in the HPLC actually refers to an aqueous buffer of 0.1% heptanesulfonic acid and 1.5% triethylamine.

### EXAMPLE 4

### Naltrexone Derivatives:

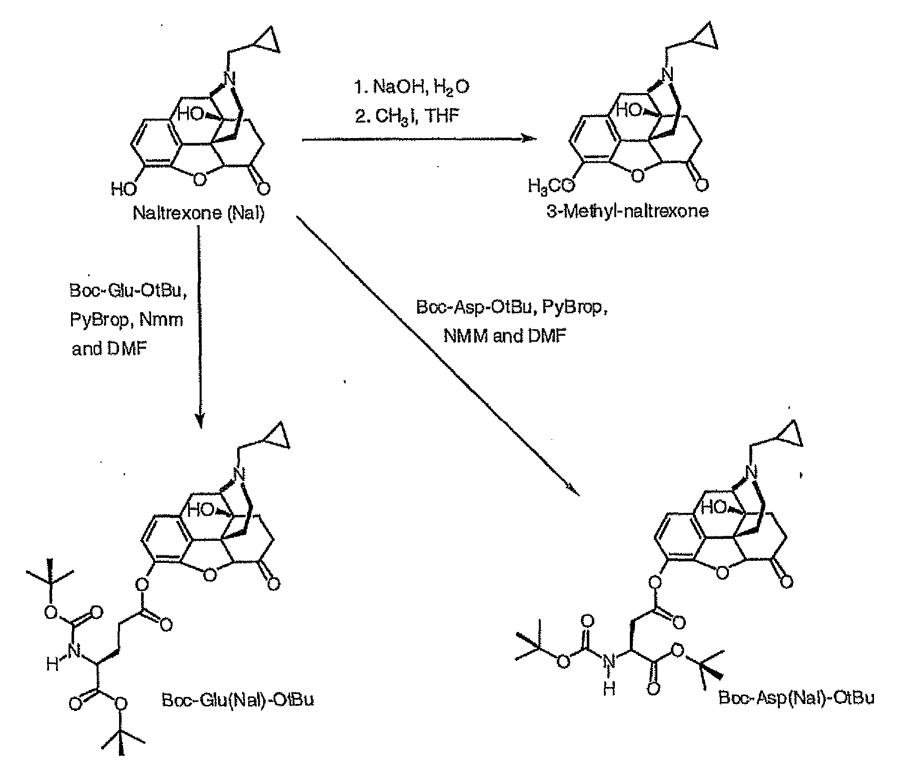

**3-Methyl-naltrexone:** Naltrexone (6.0 g, 16.5 mmol) was dissolved in 100 ml distilled water. The solution was titrated with 1N NaOH to a final pH of 11.8. In the course of the titration, neutral naltrexone precipitated from solution and then went back into solution. Upon reaching pH 11.8, the solvent was removed by rotary-evaporation under high vacuum, and the resulting solid stored under vacuum over night at room temperature.. The solid was then suspended/dissolved in anhydrous tetrahydrofuran (200 ml) and allowed to stir at room temperature under argon. A solution of iodomethane (2.1. mg, 33 mmol) in 50 ml of tetrahydrofuran was added dropwise over the course 30 minutes. The reaction was then allowed to stir an additional 3 hours at room temperature under argon. The solvent was then removed by rotary-evaporation under reduced pressure. The residual solid was then dissolved in 40 ml of CHCl₃ and the organic solution washed with 30 ml of saturated NaCl , 3x30 ml of 1N NaOH and finally twice more with 30 ml saturated aqueous NaCl. The organic solution was collected and dried over sodium sulfate. Removal of solvent by rotary-evaporation and drying over night under vacuum afforded pure 3-methylnaltrexone (5.6g, 15.8 mmol, 96% yield) as a brown residue and composition determined by TLC and ¹H-NMR. Features used to identify the compound by comparison to the spectrum of naltrexone: ¹H-NMR (360 MHz, CDCl₃) δ 6.677 (d, 1H, naltrexone aromatic), 6.591 (d, 1H, naltrexone aromatic), 3.874 (s, 3H, methoxy group.), 0.6-0.5 ppm (m, 2H, naltrexone cyclopropyl) and 0.2-0.1 ppm (m, 2H, naltrexone cyclopropyl).
**Boc-Glu(Nal)-OtBu:** The solids Boc-Glu-OtBu (0.96g, 3.18mmol), naltrexone (1.00g, 2.65mmol) and PyBrop (1.73g, 3.71mmol) were dissolved in 5 ml of anhydrous DMF and stirred at room temperature under argon. Dry N-methylmorpholine (1.08ml, 9.81mmol) was added and the reaction allowed to continue stirring at room temperature under argon. After two days additional Boc-Glu-OtBu (0.096g, 0.32mmol), PyBrop (0.173g, 0.37mmol) and N-methylmorpholine (0.10ml, 0.981mmol) were added. After 2 more days, the solvent was removed by rotary-evaporation under high vacuum. The resulting residue was then dissolved in CHCl₃, and the resulting organic solution extracted with 2x20 ml of saturated NaCl , 3x20 ml of 10% Na₂CO₃ and a final wash with 20 ml saturated aqueous NaCl. The organic solution was collected, dried over sodium sulfate and then adsorbed onto silica. Pure naltrexone conjugated amino acid (0.486g, 0.78mmol, 29%) was then isolated by flash chromatography and a gradient of 0-1.5% CH₃OH in CHCl₃. The purity of the isolated material was determined by TLC (6:1 CH₃OH/CHCl₃), and the presence of both the amino acid moiety and the naltrexone were confirmed by ¹H-NMR. Indicative protons: ¹H-NMR (360 MHz, CDCl₃) δ 6.81 (d, 1H, naltrexone aromatic), 6.63 (d, 1H, naltrexone aromatic), 4.3-4.2 (m, 1H, glutamic acid α-proton), 1.7-1.3 (pair of bs, 18H, Boc and OtBu groups.), 0.6-0.4 ppm (m, 2H, naltrexone cyclopropyl) and 0.2-0.0 ppm (m, 2H, naltrexone cyclopropyl).
**Boc-Asp(Nal)-OtBu:** Boc-Asp(Nal)-OtBu was obtained in 41% isolate yield using a similar protocol. Indicative protons: ¹H-NMR (360 MHz, CDCl₃): δ 6.84 (d, 1H, naltrexone aromatic), 6.66 (d, 1H, naltrexone aromatic), 4.6-4.5 (m, 1H, aspartic acid α-proton), 1.6-1.3 (pair of bs, 18H, Bloc and OtBu groups.), 0.7-0.5 ppm (m, 2H, naltrexone cyclopropyl) and 0.4-0.1 ppm (m, 2H, naltrexone cyclopropyl).

### NMR characterization:

While naltrexone has a complex NMR spectrum, there are several key protons that have distinct chemical shifts and are unique to naltrexone.

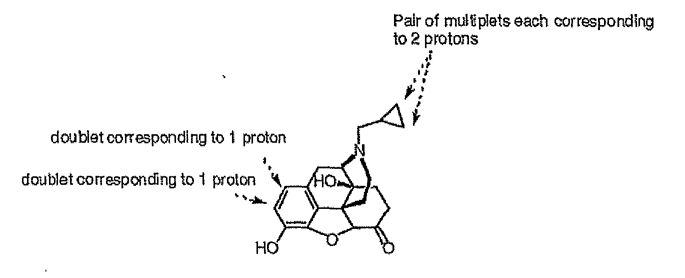

### EXAMPLE 5

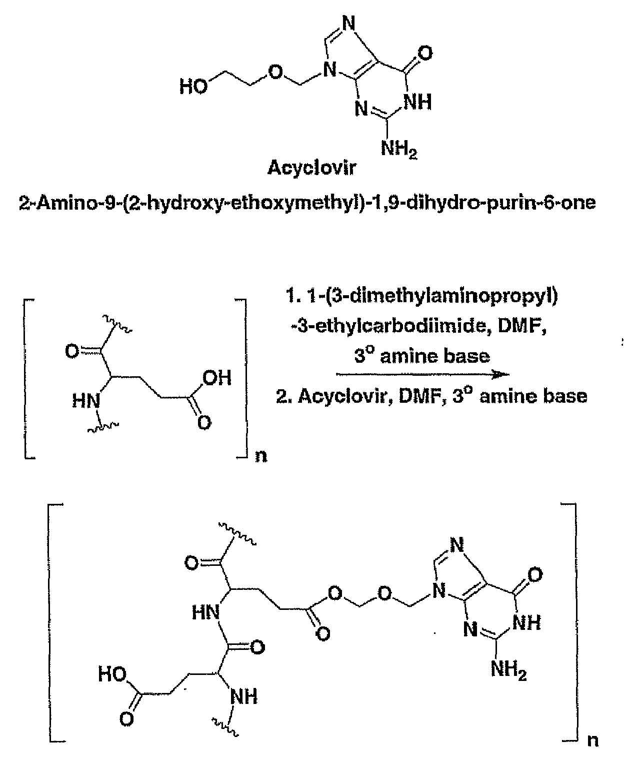

### Poly-Glu(Acyclovir)

To a solution of poly-glu₁₅ (0.600g, 0.310mmol) in DMF (25ml) was added EDCI (2.07g, 10.8mmol). The resulting mixture was allowed to stir at ambient temperature for one hour. Then, N-methyl morpholine (0.51ml, 4.7mmol) was added followed by a mixture of acyclovir (1.74g, 7.75mmol), DMF (25ml) and N-ethyl morpholine (0.85ml). The reaction mixture was stirred at ambient temperature for 4 days. After this, water (50ml) was added and all solvent was removed. To the dried mixture was added water (100ml) and a precipitate of unreacted acyclovir formed. Solid was centrifuged and the supernate was purified using ultrafiltration (YM1 membrane). Approximately 300ml water was allowed to pass through the membrane. NMR has shown an unexpected alkyl-urea side chain attached impurity. Poly-glu(acyclovir) (0.970g) was obtained as a light yellow solid: ¹H NMR (D₂O) δ 1.11 (br m, 4H, urea), 2.01 (br m, 2H, Glu-β H), 2.39 (br m, 2H, Glu-γH), 2.72 (br m, 2H, urea), 3.32 (br m, 6H, acyclovir CH₂ and urea), 3.83 (br m, 3H, urea), 4.38 (br d, 3H, Glu-α H), 5.47 (br s, 2H, acyclovir 1' CH2), 7.94 (br s, 1H, acyclovir 8 CH).

### EXAMPLE 6

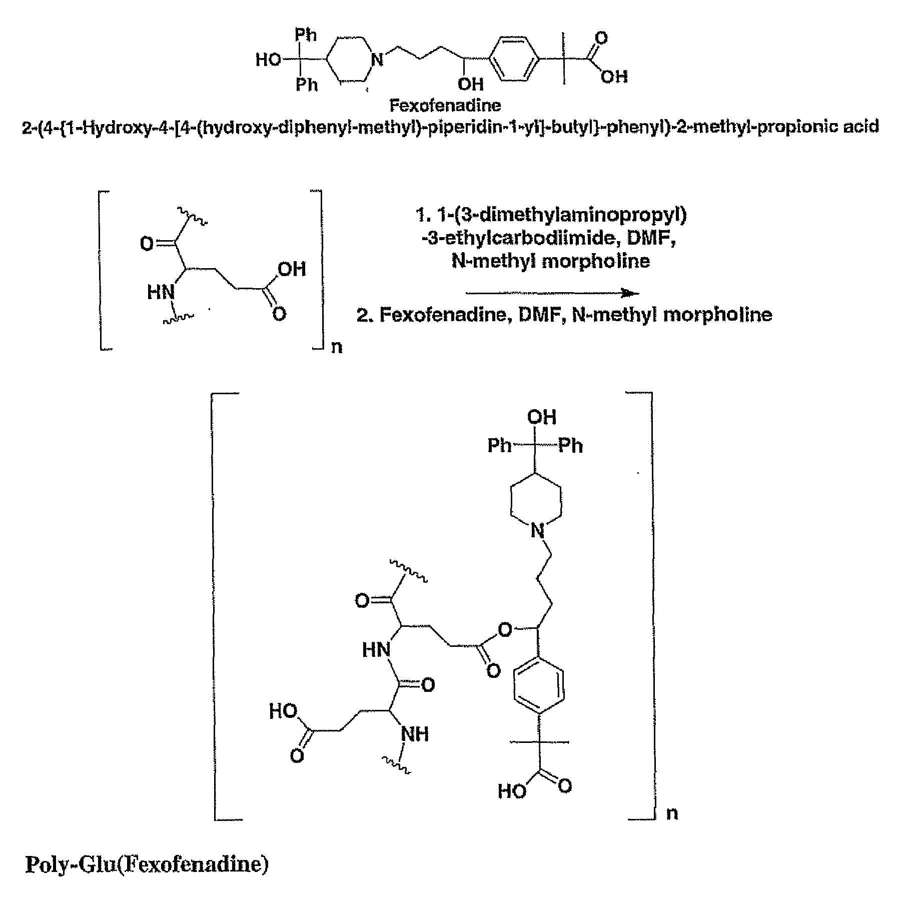

To a solution of poly-glu₁₅ (0.078g, 0.040mmol) in DMF (5ml) was added EDCI (0.035g, 0.18mmol). After sting for 30 minutes, N-methyl morpholine was added (0.03ml, 0.24mmol). After stirring for 10 minutes, a solution of fexofenadine (0.100g, 0.20mmol), N-methyl morpholine (0.07ml, 0.60mmol) and DMF (5ml) was added via a syringe. After stirring reaction at ambient temperatures for three days, sample was dissolved in water (25ml). A solid precipitate formed which was both drug-conjugate and free fexofenadine. Water was acidified and all solids dissolved. Purification using ultrafiltration (YM1 followed by YM3) and size exclusion chromatography using Sephadex-25 at pH 7 yielded poly-glu(fexofenadine) (0.010g) as a white solid: ¹H NMR (D₂O) δ 1.37 (s, 8H, fex. CH₂ and CH₃), 1.58 (br m, 5H, fex. CH and CH₂), 1.99 (br m, 24H, Glu-β H), 2.31 (br m, 24H, Glu-γ H), 2.70 (br m, 10H, fex. CH and CH₂), 4.14 (br m, 26H, Glu-α H), 7.25 (br m, 14H, fex. aromatic H).

### EXAMPLE 7

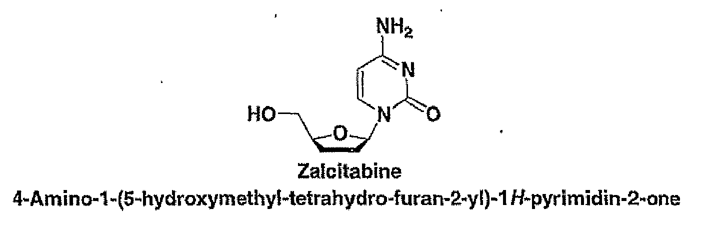

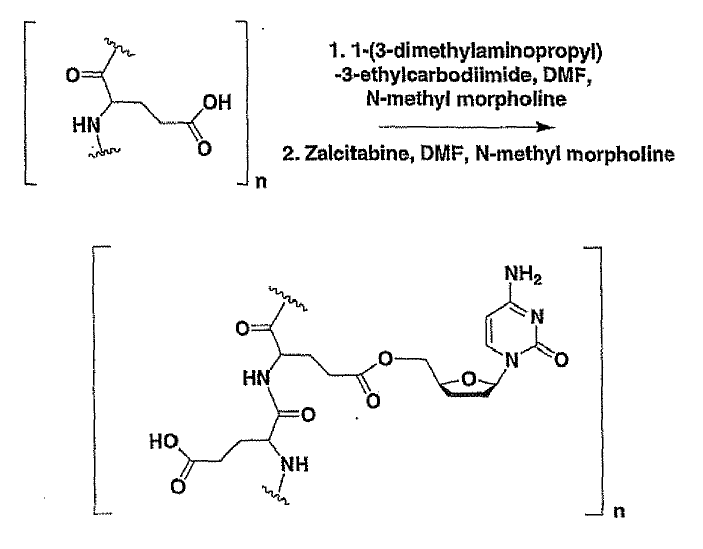

### Poly-Glu(Zalcitabine)

To a solution of poly-glu₁₅ (0.123g, 0.060mmol) in DMF (8ml) was added EDCI (0.403g, 2.10mmol). After 30 minutes, N-methyl morpholine (0.13ml, 1.2mmol) was added. After 35 minutes, a solution of zalcitabine (0.200g, 0.95mmol), N-methyl morpholine (0.10ml, 0.9mmol) and DMF (2ml) was added via a syringe. The resulting mixture was stirred at ambient temperature for 48 hours. Solvent was removed and the residue was dissolved in water (15ml). Ultrafiltration (YM1 followed with YM3) and size exclusion using Sephadex-25 at pH 7 yielded poly-glu(zalcitabine) (0.083g) as a light yellow solid: ¹H NMR (DMSO-d₆ w/D₂O) δ 1.14 (br m, 20H, urea), 1.90 (br m, 30H, Glu-β H, Glu-γ H and CH₂ in zalcitabine), 2.66 (br m, 4H, urea), 3.24 (br m, 36H, urea, CH and CH₂ in zalcitabine), 4.29 (br m, 8H, Glu-α H), 5.87 (br s, 1H, zalcitabine 1' CH), 7.18 (br s, 1.19H, zalcitabine NH₂), 8.52 (br s, 1H, zalcitabine 6 CH).

### EXAMPLE 8

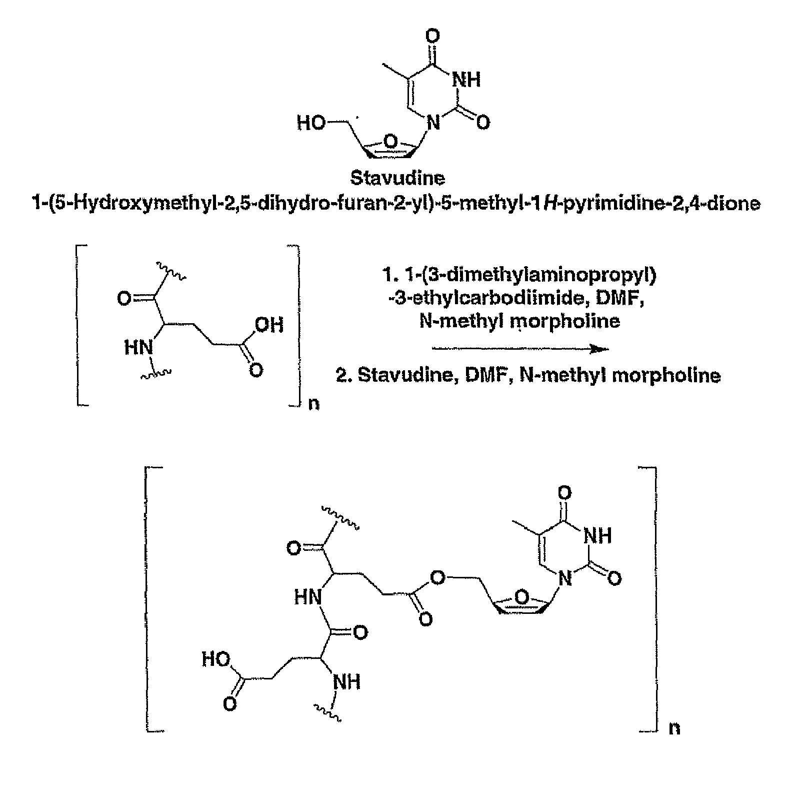

### Poly-Glu(Stavudine)

Preparation was similar to poly-glu(zalcitabine). Purification using ultrafiltration (YM1) yielded poly-glu(stavudine) (0.089g) as a white solid: ¹H NMR (D₂O) δ 1.87 (s, 3H, stavudine 5 CH₃), 2.06 (br m, 38H, Glu-β H and Glu-γ H), 2.49 (br m, 12H, Glu-γ H), 3.75 (br m, 12H, urea and stavudine 5' CH₂), 3.96 (br m, 12H, urea), 4.45 (br d, 13H, Glu-α H), 5.98 (d, 1H, stavudine 1' CH), 6.48 (d, 1H, stavudine 3' CH), 6.96 (d, 1H, stavudine 2' CH), 7.63 (s, 1H, stavudine 6 CH).

### EXAMPLE 9

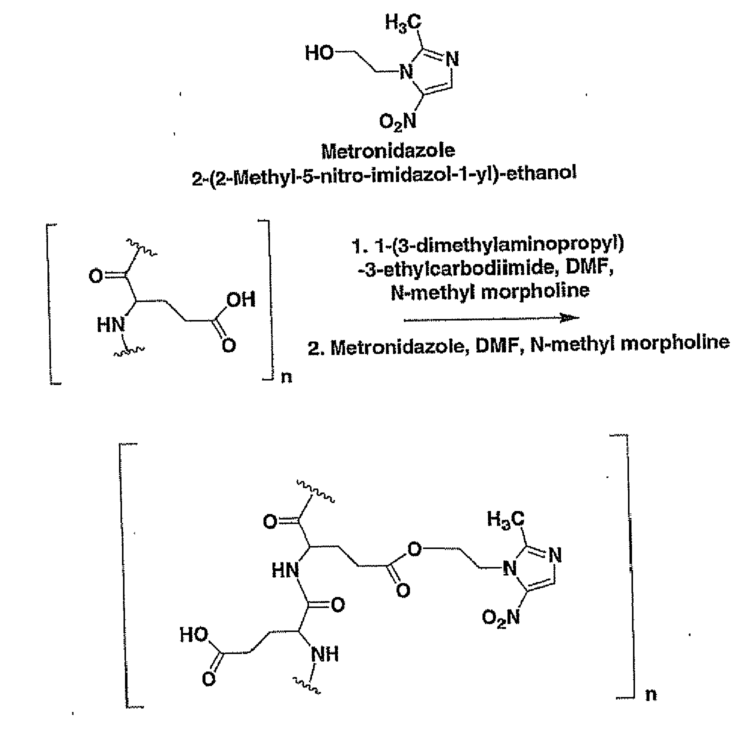

### Poly-Glu(Metronidazole)

Preparation was similar to poly-glu(zalcitabine). Purification using ultrafiltration (YM1) yielded poly-glu(metronidazole) (0.326g) as a yellow solid: ¹H NMR (DMSO-d₆) δ 1.18 (br d, 13H, urea), 1.93 (br s, 17H, Glu-β H and Glu-γ H), 2.71 (br s, 16H, urea), 4.01 (br m, 18H, Glu-α H and metronidazole CH₂), 4.58 (br s, 2H, metronidazole CH₂), 8.05 (br s, 1H, metronidazole 2 CH).

### EXAMPLE 10

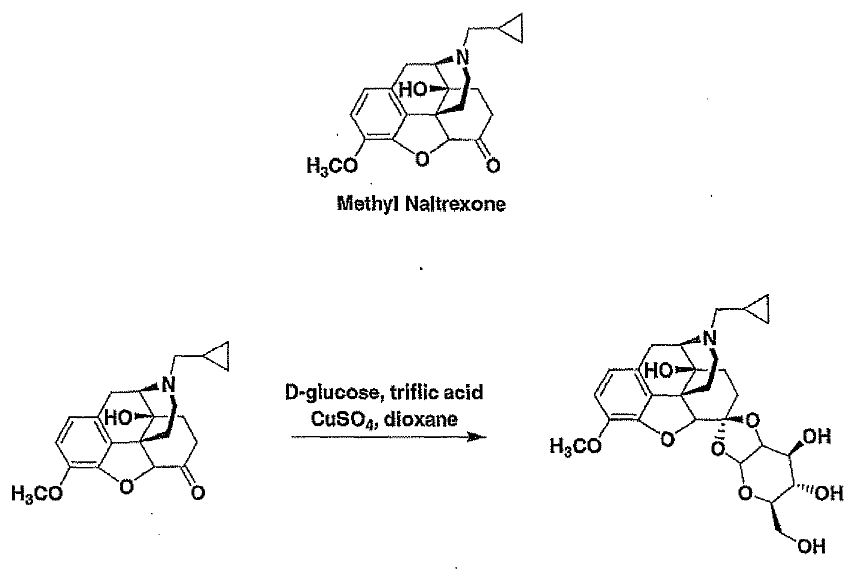

### Methyl Naltrexone - Glucose Ketal Conjugate

To a solution of methyl naltrexone (0.200g, 0.56mmol) in dioxane (20ml) was added D-α-glucose (2.02g, 11.2mmol), triflic acid (0.05ml, 0.62mmol), and CuSO₄, (1.00g). The reaction mixture was stirred at ambient temperatures for 4 days. Reaction was then filtered, neutralized with NaHCO₃ (sat.) and filtered again. Dioxane and water were removed and the residue was taken up in CHCl₃ and extracted with water (3X100ml). The organic layer was dried over MgSO₄ and solvents were removed under reduced pressure. Crude product was purified over silica gel (0-10% MeOH in CHCl₃) to obtain the ketal conjugate (0.010g) in a 1:1 mixture with free methyl naltrexone: ¹H NMR (CDCl₃) δ 0.14 (br s, 4H, naltrexone cyclopropyl), 0.53 (br m, 4H, naltrexone cyclopropyl), 0.90 (m, 2H, naltrexone cyclopropyl), 1.48 (m, 6H, naltrexone), 2.19-2.78 (m, 12H, naltrexone), 3.03 (m, 2H, naltrexone), 3.75 (q, 2H, glucose), 3.87 (m, 8H, naltrexone CH₃ and glucose), 3.97 (q, 2H, glucose), 4.14 (q, 1H, glucose), 4.33 (t, 1H, glucose), 4.66 (s, 1H, naltrexone), 6.65 (m, 4H, naltrexone).

### EXAMPLE 11

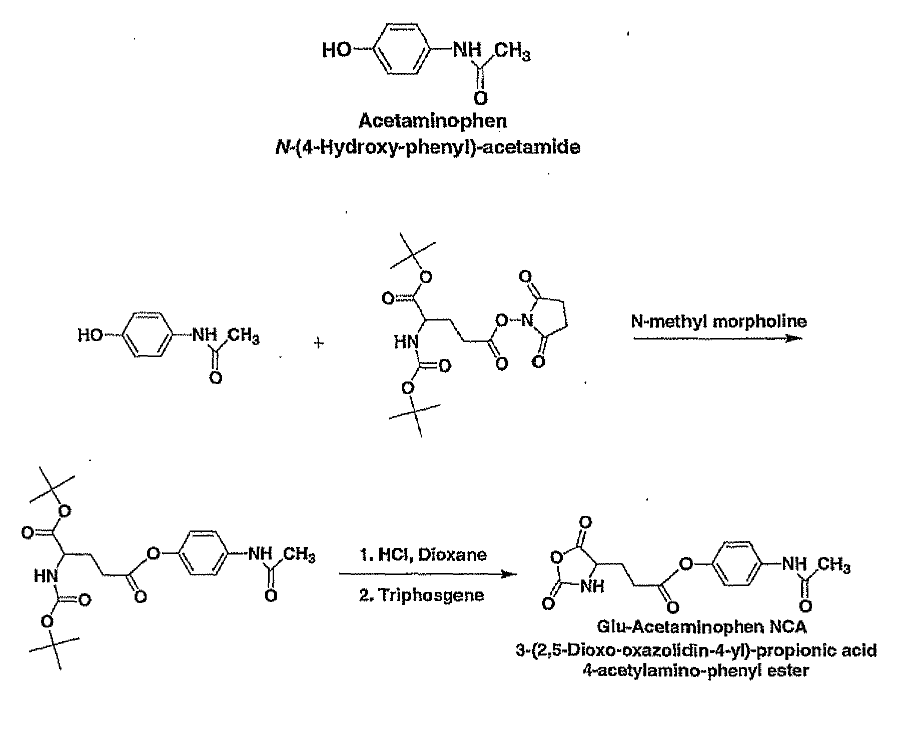

### 2-Amino-pentanedioic acid 5-(4-acetylamino-phenyl) ester or Glu(Acetaminophen)

To a solution of Boc-Glu(OSuc)-OtBu (0.500g, 1.25mmol) and acetaminophen (0.944g, 6.25mmol) in THF (15ml) was added N-methyl morpholine (1.40ml, 12.5mmol). The reaction was allowed to heat to reflux and stirred at reflux overnight. Solvent was then removed and the crude compound was purified over silica gel (50-75% ethyl acetate in hexanes) to obtain Boc-Glu(Acetaminophen)-OtBu (0.432g, 0.900mmol, 72%): ¹H NMR (CDCl₃) δ 1.43 (d, 18H, t-Bu), 1.97 (m, 1H, Glu-β H), 2.12 (s, 3H, acetaminophen CH₃, 2.25 (m, 1H, Glu-β H), 2.60 (m, 2H, Glu-γ H), 4.25 (m, 1H, Glu-α H), 7.04 (d, 2H, acetaminophen aromatic), 7.48 (d, 2H, acetaminophen aromatic).

A solution of Boc-Glu(Acetaminophen)-OtBu (0.097g, 0.20mmol) in 4N HCl in dioxane (10ml) was stirred at ambient temperatures for 2 hours. Solvent was removed to obtain glu(acetaminophen) (0.90g) as the HCl salt: ¹H NMR (D₂O) δ 2.19 (s, 3H, acetaminophen CH₃), 2.41 (m, 2H, Glu-β H), 2.97 (t, 2H, Glu-γ H), 4.18 (t, 1H, Glu-α H), 7.19 (d, 2H, acetaminophen aromatic), 7.51 (d, 2H, acetammophen aromatic); ¹³C NMR (DMSO) δ 23.80, 29.25, 51.00, 66.24, 119.68, 121.69, 137.00, 145.35, 169.23, 170.42, 170.79.

### 3-(2,5-Dioxo-oxazoildin-4-yl)-propionic acid 4-acetylaminophenyl ester or Glu(Acetaminophen) NCA

To a mixture of 2-amino-pentanedioic acid 5-(4-acetylamino-phenyl) ester (1.54g, 4.29mmol) in THF (40ml) was added triphosgene (1.02g, 3.43mmol). The resulting solution was stirred at reflux for 3 hours. During reaction, the product precipitated and was filtered away to obtain the NCA of glu(acetaminophen) (1.02g, 2.64mmol, 62%) as an off white solid: ¹H NMR (DMSO-d₆) δ 2.01 (s, 3H, acetaminophen CH₃), 2.15 (m, 2H, Glu-β H), 2.81 (m, 2H, Glu-γ H), 3.76 (t, 1H, Glu-α H), 7.06 (d, 2H, acetaminophen aromatic), 7.63 (d, 2H, acetaminophen aromatic), 8.57 (br s, 1H, amide), 10.19 (s, 1H, amide); ¹³C NMR (DMSO) δ 23.81, 29.25, 52,13, 54.62, 119.66, 121.71, 136.98, 145.35, 167.44, 168.19, 170.46, 170.77.

### EXAMPLE 12

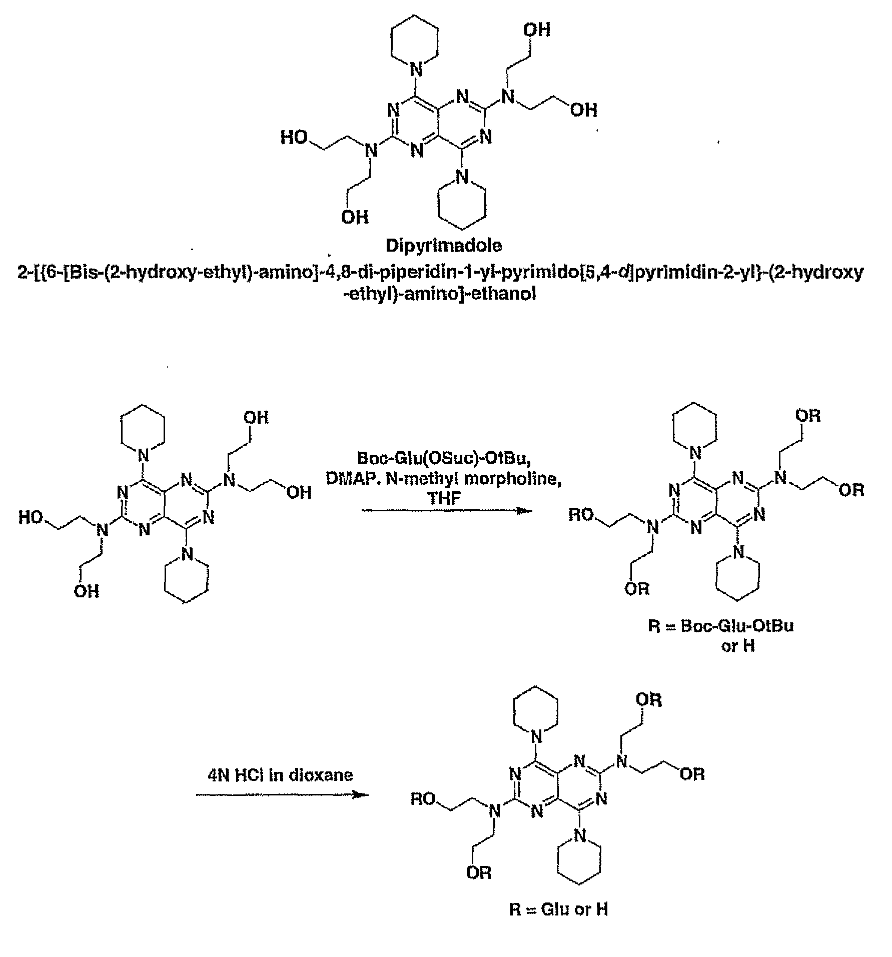

### Glu(Dipyrimadole)

To a solution of dipyrimadole (0.500g, 0.990mmol) and Boc-Glu(OSuc)-OtBu (3.96g, 9.91mmol) in THF (35ml) was added DMAP (0.072g, 0.60mmol) and N-methyl morpholine (0.22ml, 1.98mmol). The solution was then reflexed for 48 hours. Solvent was then removed and crude product was purified over silica gel (25-50% ethyl acetate in hexanes). Two major products were isolated, one with R=2-3 (0.57g) and another with R=3-4 (2.80g), as bright yellow oils: [for R=2-3 ¹H NMR (CDCl₃) δ 1.41 (s, 42H, t-Bu), 1.64 (br s, 5H, dipyrimadole), 1.85 (m, 2H, Glu-β H), 2.07 (m, 2H, Glu-β H), 2.37 (m, 4H, Glu-γ H), 3.60-4.24 (m, 12H, Glu-α H and dipyrimadole)]; [for R=3-4 similar as above except 1.44 (s, 56H, t-Bu)].

A solution of Boc-Glu(dipyrimadole)-OtBu (R=2-3, 0.57g) and 4N HCl in dioxane (20ml) was stirred at ambient temperature for 2.5 hours. Solvent was removed and the product (0.280g) was a bright yellow solid: ¹H NMR (DMSO-d₆) δ 1.65 (br m, 4H, Glu-β H and dipyrimadole), 2.04 (br m, 2H, Glu-β H), 2.40 (br m, 4H, Glu-γ H), 3.75 (br m, 8H, dipyrimadole), 3.91 (br m, 2H, Glu-α H), 8.55 (br m, 2H, amide H).

### EXAMPLE 13

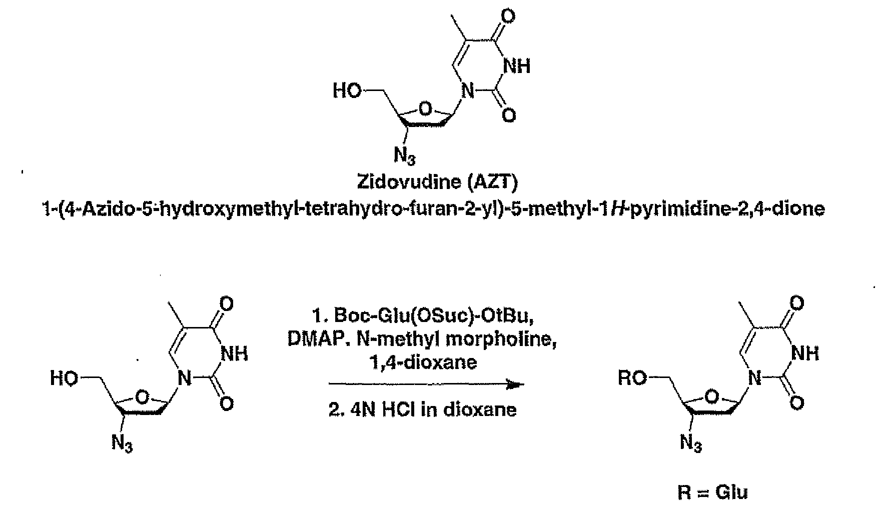

### Glu(AZT)

To a solution of zidovudine (1.00g, 3.75mmol) and Boc-Glu(OSuc)-OtBu (3.00g, 7.49mmol) in dioxane (75ml) was added DMAP (0.137g, 1.13mmol) and N-methyl morpholine (0.82ml, 7.49mmol). The solution was heated to reflux for 6 hours and heated at 70°C for 12 hours. Solvent was then removed and the crude product was purified over silica gel (100%CHCl₃) to obtain Boc-Glu(AZT)-OtBu (1.09g, 1.91mmol, 51%) as a yellow foam: ¹H NMR (CDCl₃) δ 1.40 (d, 32H, t-Bu), 1.86 (s, 3H, AZT CH₃), 2.11 (m, 2H, Glu-β H), 2.38 (m, 4H, Glu-γ H and AZT 2' CH₂), 4.00-4.31 (m, 4H, AZT 4' CH, 5' CH₂ and Glu-α H), 5.21 (d, 1H, AZT 3' CH), 6.01 (t, 1H, AZT 1'CH), 7.16 (s, 1H, AZT 6 CH).
A solution of Boc-Glu(AZT)-OtBu (1.09g, 1.91mmol) in 4N HCl in dioxane (20ml) was stirred for 4 hours and solvent removed. The product, Glu(AZT) (0.89g, 1.99mmol, quant.), was obtained as a yellow glass: ¹H NMR (D₂O) δ 1.89 (s. 3H, AZT CH₃), 2.21 (m, 4H, Glu-β H and AZT 2' CH₂), 2.58 (m, 2H, Glu-γ H), 3.70 (t, 1H, Glu-α H), 4.05-4.41 (m, 4H, AZT 4' CH, 3' CH and 5' CH₂), 6.18 (t, 1H, AZT 1' CH), 7.51 (s, 1H, AZT 6 CH).

### EXAMPLE 14

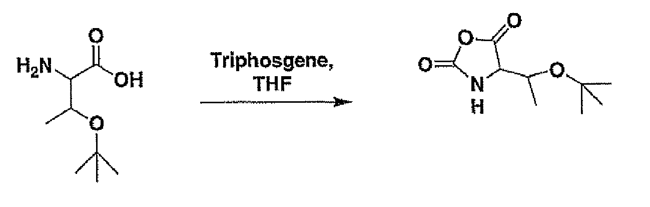

### Threonine NCA

To a mixture of Thr-OtBu (0.500g, 2.85mmol) in THF (25ml) was added triphosgene (0.677g, 2.28mmol). The resulting solution was stirred at reflux for 3 hours. The solution was evaporated to dryness to obtain Thr-NCA (0.500g, 2.48mmol, 87%) as a white solid. Thr-NCA was used without further characterization.

### EXAMPLE 15

### Preparation of a DRUG-GLU conjugate as a starting synthon for polymerization

With non-primary amine drug candidates, formation of the Drug-poly-Glu conjugate may prove problematic. To overcome this difficulty, the following scheme was used, wherein the drug is first conjugated to Glu, and this synthon is then used to initiated coupling. The protocol has been successfully applied to sertraline and to metoclopramide.

### Protocol for coupling Boc-Glu(OtBu)-OH to Sertraline

1. Boc-Glu(OtBu)-OH (0.44 g, 1.46 mmol) and PyBOP (0.84 g, 1.60 mmol) were dissolved in dry DMF (15 mL) with stirring.
2. DIEA (0.31 mL, 1.75 mmol) was added and the amino acid derivative was allowed to activate for 15 minutes.
3. Sertraline hydrochloride (0.50 g, 1.46 mmol) was added to the stirring mixture followed by an additional 0.31 mL DIEA.
4. The mixture was allowed to stir for 16 h.
5. The solution was stripped yielding a brown oil.
6. The oil was dissolved in EtOAc (100 mL) and the resulting solution was washed with 10 % HCl (3 x 30 mL), saturated NaHCO₃, 4M NaHSO₄, and brine (2 x 30 mL, respectively).
7. The solution was dried over MgSO₄, filtered and the solvent was removed by rotary evaporation under reduced pressure, yielding a light brown oil.
8. The oil was dried on the vacuum manifold and the product was purified by column chromatography on silica gel using EtOAc/Hexanes 1:5 to 1:4 solvent system.
9. The product fractions were pooled and solvent was again removed by rotary evaporation yielding 0.85 g (99%) of the final product, Sertraline-NH-C(O)-Glu-NH3+.
10. The preparation was dried on the vacuum manifold.

### EXAMPLE 16

### Synthesis of Poly-Lysine-Ibuprofen

I. Preparation of Ibuprofen-O-Succinimide (RI-172) (Grafe & Hoffman, Pharmazie 55: 286-292, 2000) 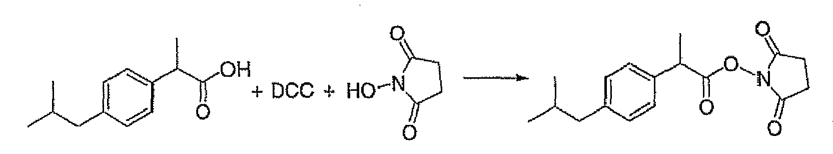 To a stirring solution of ibuprofen (2.06 g, 10 mmol) in 5 mL of dioxane at room temperature was added a solution of dicyclohexylcarbodiimide (DCC, 2.27 g, 11 mmol) in 25 mL of dioxane. After 10 minutes a solution of N-hydroxysuccinimide (NHS, 1.16 g, 10 mmol) in 15 mL of dioxane was added. The reaction mixture was allowed to stir at room temperature for 5 hours and then filtered through a sintered glass funnel to remove the dicyclohexylurea (DCU). After rotary evaporation, the product was crystallized from methylene chloride/hexanes to yield 2.36 g (78%) of a colorless solid. ¹H-NMR (dmso-d6): δ 0.86 (d, 6, CH₃), 1.49 (d, 3,α- CH₃), 1.81 (m, 1, CH), 2.43 (d, 2, CH₂), 3.33 (m, 4, CH₂CH₂), 4.22 (q, 1, CH), 7.16 (d, 2, ArH), 7.28 (d, s, ArH).
II. Conjugation of Poly-Lysine with Ibuprofen-O-Succinimde (RI-197) 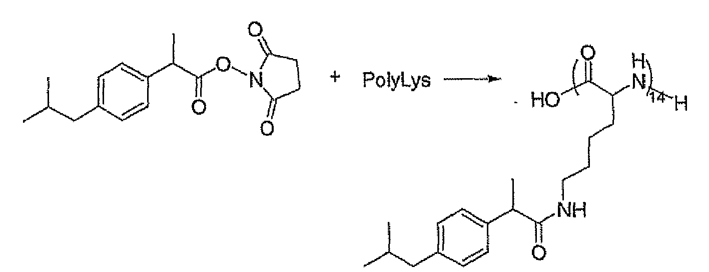 Poly-lysine-HBr (Sigma, 100 mg, 34.5mmol) was dissolved in 1 mL of water that had brought to a pH of 8 with sodium bicarbonate, and stirred at room temperature. To this solution was added a solution of ibuprofen-O-succimmide (116 mg, 380 nmol) in 2 mL of dioxane. After stirring overnight, the dioxane was removed by rotary evaporation and diluted with 10 mL of pH 8 sodium bicarbonate in water. The precipitated product was filtered through a sintered glass funnel and washed with 3 X 10 mL of water and 4 X 10 mL of diethyl ether. After drying overnight by high vacuum the solid product was scraped out yielding 105 mg (62%). ¹H-NMR (dmso-d6): δ 0.85 (br s, 6, CH₃), 1.27 (br s, 3,α- CH₃), 1.40-1.79 (m, 5, CH of ibu and lysine γ and δ CH₂CH₂), 2.31 (d, 2, β CH₂), 2.41-2.52, under dmso (m, 2, β CH₂), 2.73-3.01 (m, 2, ε CH₂), 3.51-3.85 (m, 1 ibu CH), 4.01-4.43 (m, 1, α CH), 7.14 (d, 2, ArH), 7.6 (d, 2, ArH), 7.90-8.06 (m, 2, NH).

### EXAMPLE 17

### Summary of the synthesis of [Lysine]ₓₓ -[Gemfibrozil or Naproxen] or [Glu]ₓₓ L-DOPA

### Synthesis of [Glu]_{15 -} L-dihyciraxyphenylalanine or [Glu]₁₅-L-DOPA

L-DOPA (0.050 g, 254 □mol) and GluNCA (0.666 g, 3.85 mmol) were dissolved in 6 ml DMF. After stirring overnight under Argon, the reaction was examined by thin layer chromatography (9:1 H₂O: HOAc) showed some free drug (R_{f}= 0.70) and a more polar spot presumed to be polymer (R_{f}= 0.27). The reaction was quenched by the addition of 12 ml H₂O. The pH was adjusted to pH 1-2 using IN HCl. The solvent was removed by rotary evaporation and the viscous residue dried in vacuum. The resultant syrup was transferred to a new vessel in H₂O and lyophilized. The resulting crystals were off white to light brown. Yield: 0.470 g, 62%. ¹H NMR showed pyroglutamic acid contamination; therefore, the material was suspended in H₂O and ultrafiltered (Millipore, regenerated cellulose, YM1, NMWL =1000), and the retentate dried under vacuum. Yield: 0.298 grams. ¹H NMR (500MHz, DMSO) indicated a relative ratio of 30:1 Glu:L-DOPA, 6.6 (L-DOPA aromatic), 6.4 (L-DOPA aromatic), 4.1 (Glu, α)
1.85 (Glu, β), 2.25 (Glu, γ, L-DOPA), 2.3 (L-DOPA, benzylic), 12.4-11.5 (Glu, CO₂H), 8.0 (Glu, amide)

### Synthesis of [Glu]₁₀ -L-DOPA

As in the synthesis of [Glu]₁₅-L-DOPA except 0.439 grams of GluNCA were used. The final yield of purified material was 0.007 grams.
The ¹H NMR (500MHz, DMSO) indicates 8:1 Glu:L-DOPA.

### Synthesis of Naproxen-Succinimide

To Naproxen (2.303 g, 10 mmol) in 5 ml of dioxane was added N-hydroxysuccinimide (1.16 g, 10 mmol) dissolved in 15 ml of dioxane and dicyclohexylcarbodiimide (2.27 g, 11mmol) in 25 ml of dioxane. The reaction was stirred overnight and the insoluble dicyclohexylurea removed by filtration. The solvent was removed by rotary evaporation and the residue dissolved in 30-40 ml CH₂Cl₂. Approximately 10 ml hexane was added and the mixture was chilled to 4°C for 2 hr. Additional hexane was added dropwise until small planar white crystals began to form and the solution was refrigerated overnight. The activated ester was harvested, washed with hexane and dried in vacuum (2.30 g, 70.0 %): ¹H NMR (500MHz, DMSO) 1.70 (d, 3H, CH₃) 2.9 (s, 4H, succinimide), 3.91 (s, 3H, OCH₃), 4.1.8 (q, 1H, methane) 7.75-7.12 (m, 6H, aromatic).

### Synthesis of polylysine-Naproxen

To [Lys]₁₄ · 14 · HBr (0.100 g, 35 mmol) in 1 ml of H₂O (containing 10 mg/ml Na₂CO₃) was added Naproxen-Succinimide (0.124 g, 379 mmol) in 2 ml of dioxane. After stirring overnight a precipitate formed. More precipitate was formed by the addition of 30-40 ml of H₂O (containing 10 mg/ml Na₂CO₃), isolated by filtration and washed with 50 ml of Et₂O. The fine white powder was dried (0.095 g, 53%): ¹H NMR (500NMR, DMSO) 8.1 (m, 1H, lysine; amide), 7.8-7.4 (m, 6H, aromatic), 4.4-4.1 (m, 2H, α methine), 3.3 (s, 3H, OCH₃), 2.8 (m, 2H, ε), 1.7-1.0 (m, 9H, β, γ, δ, CH₃).

### Synthesis of Gemfibrozil - Succinimide

To Gemfibrozil (GEM) (5.0 g, 20.0 mmol) in 30 ml dioxane was added N- hydroxysuccinimide (2.3 g, 20.0 mmol) in 20 ml dioxane and dicyclohexylcarbodiimide (4.5 g, 22.0 mmol) in 50 ml dioxane. The reaction was stirred overnight and the insoluble dicyclohexylurea removed by filtration. The solvent was removed by rotary evaporation and the residue dissolved in 15 - 20 ml of CH₂Cl₂. Hexane was added dropwise until crystal formation was seen and the mixture was chilled to 4° C overnight. Approximately 3 ml of additional n-hexane was added and the mixture chilled to -20° C overnight. The activated ester formed small planer crystals and was harvested, washed with hexane and dried in vacuum (5.8 g, 80%): ¹H NMR (500 MHz, DMSO) 1.2, 1.3 (s, 6H, CH₃), 1.8-1.5 (m, 6H, GEM CH₂). 2.3-2.1 (s, 6H, aromatic CH₃) 2.85-2.7 (d, 4H, succimmide CH₂), 7.0-6.6 (m, 3H, aromatic).

### Synthesis of polylysine-Gemfibrozil

To [Lys]₁₁^{·}11^{·}HBr (0.100 g, 43.5 αmol) in 1 ml of H₂O (containing 10 mg/ml Na₂CO₃) was added Gemfibrozil-sucrinimide (0.094 g, 261.1 αmol) in 2 ml dioxane. After stirring overnight a precipitate formed. More precipitate was formed by the addition of 30 ml of H₂O (containing 10 mg/ml Na₂C0₃), isolated and washed with 50 ml Et₂O. The fine white powder was dried (0.019 g, 1 %): ¹H NMR (500MHz, DMSO) 1.5-1.0 (m, 12H, β, γ, δ, CH₃), 1.85-1.5 (m, 4H, CH₂), 2.3,2.1 (s, 6H, aromatic CH₃), 3.35 (s, 2H, ε), 3.85 (s, 2H, OCH₂), 4.05 (s, 1H, α), 5.6 (d,1H, carbamate), 7.0-6.7 (m, 3H, aromatic), 8.0 (d, 1H, amide).

### EXAMPLE 18

All reagents were used as received. ¹H NMR was run on a Bruker 300 MHz (300) or JEOL 500 MHz (500) NMR spectrophotometer using tetramethylsilane as an internal standard. Thin layer chromatography was performed using plates precoated with silica gel 60 F₂₅₄. Flash chromatography was performed using silica gel 60 (230-400 mesh).

### Preparation of polyArg

### Method 1

To H-Arg(Z) ₂-OH (0.300 g, 0.68 mmol) in 3.0 ml dry DMSO was added diphenylphosphorylazide (219 µl, 1.02 mmol) and triethylamine (236 µl, 1.69 mmol). The reaction was stirred for 48 h under Ar upon which the solution was poured into 100 ml H₂O. The resulting heterogeneous solution was centrifuged to isolate the white precipitate which was washed 3 x 100 ml H₂O, 3 x 100 ml CH₂OH and 100 Et₂O and then vacuumed dried to obtain 172 mg of an off white solid: ¹H NMR (500 MHz, DMSO) 7.31 (m, 10H), 5.21 (m,1H, benzylic), 5.01 (m, 1H, benzylic), 3.83 (m, 1H, α), 3.34 (m, 2H, δ) 1.54 (m, 4H, β, γ).

This material was dissolved in 1.5 ml dry anisole and stirred with 0.3 ml anhydrous methanesulfonic acid for 3 h upon which another 0.3 ml anhydrous methanesulfonic acid was added and the solution stirred for 1 h. The reaction mixture was poured into 6 ml Et₂O and refrigerated for 15 m. The heterogeneous biphasic mixture was concentrated to 0.5 ml by rotary evaporation. Thrice, an additional 8 ml Et₂O was added and the biphasic mixture centrifuged and the supernatant removed leaving a yellowish gum. This residue was washed twice with 6 ml acetone, centrifuged and the supernatant discarded leaving behind a white-yellow residue. The residue was dissolved in 0.3 ml H₂O and shaken with Amberlite IRA-400. The resin was removed by filtration and washed with 3 ml H₂O. The combined eluent and wash were dried in vacuum yielding a yellow film 0.063 g, (90% yield): ¹H NMR (500 MHz, D₂O) 4.37 (m, 1H, α), 3.22 (m, 2H, δ) 1.94-1.66 (m, 4H, β, γ); MALDI-MS shows a degree of polymerization varying between six to fourteen residues.

### Method 2

To Boc-Arg(Z) ₂-OH (0.025 g, 0.05 mmol) and H-Arg(Z) ₂-OH (0.280 g, 0.63 mmol) in 3.0 ml dry DMSO was added diphenylphosphorylazide (219 µl, 1.02 mmol) and triethylamine (236 µl, 1.69 mmol). The reaction was stirred for 48 h and then poured into 100 ml H₂O. The heterogeneous solution was centrifuged and the precipitate washed 3 x 100 ml H₂O, 3 x 100 ml CH₃OH and 100 Et₂O and then vacuumed dried to obtain 132 mg of solid: ¹H NMR (500 MHz, DMSO) 7.31 (m, 10H), 5.21 (m, 1H, benzylic), 5.01 (m, 1H, benzylic), 3.83 (m, 1H, α), 3.34 (m, 2H, δ) 1.54 (m, 4H, β, γ).

The protected polymer was dissolved in 1.5 ml dry anisole and stirred with 1.3 ml anhyd methanesulfonic acid for 4 h. The solution was concentrated to 0.5 ml by rotary evaporation. Et₂O (8 ml) was added and the biphasic system centrifuged and the supernatant discarded. Thrice, 10 ml acetone was added, the solution centrifuged and the supernatant discarded. The pellet was dried overnight in vacuum and then dissolved in 0.3 ml H₂O and shaken with Amberlite IRA-400. The resin was removed by filtration and washed with 3 ml H₂O. The combined eluent and wash were dried in vacuum yielding a yellow film 0.019, (24% yield); ¹H NMR (500 MHz, D₂O) 4.37 (m, 1H, α), 3.22 (m, 2H, δ) 1.94-1.66 (m, 4H, β, γ); MALDI-MS shows a degree of polymerization varying between five to eleven residues.

### Preparation of T4 Conjugates

T4 conjugated to aminoacid polymers were either prepared by coupling (protected) T4 to commercially available aminoacid homopolymers or incorporated by polymerization of a T4 moiety with the corresponding N-carboxyanhydride aminoacid.

### T4 Conjugation to preformed homopolymer

To N-TeocT4 (0.017 g, 17 µmol) in 1 ml dry DMF was added dicyclohexylcarbodiimide (0.004 g, 18 µmol). After stirring for 30 minutes N-dimethyl-4-aminopyridine (0.004 g, 36 µmol) and Gly₁₈ (0.017 g, 17 µmol) were added and the reaction stirred overnight. The cloudy solution was poured into 20 ml H₂O and extracted twice with 10 ml CH₂Cl₂. The aqueous component was acidified to pH 3 with 1 N HCl and chilled to 4° C. The material was isolated by centrifugation and the pellet thrice washed with 8 ml H₂O. The pellet was dried in vacuum to yield dicyclohexylurea and N-TeocT4-Gly₁₈: ¹H NMR (500 DMSO) 7.8 (T4 aromatic), 7.1 (T4 aromatic), 4.1 (α).

To the impure protected polymer was added 2 ml trifluoroacetic acid. The reaction was stirred for 2 h and the solvent removed by rotary evaporation. The residue was dissolved in 1 ml DMF and the insoluble material removed by filtration. The DMF was removed by rotary evaporation and dried in vacuum to yield a white material (.012 g, 40%): ¹H NMR (500 DMSO) 7.75 (T4 aromatic), 7.08 (T4 aromatic), 4.11 (bs, α).

### Preparation of aminoacid NCA.

To the L-aminoacid (1.5 g) in 100 ml dry THF was added triphosgene (0.8 eqv). The reaction was vessel was equipped with a reflux condenser and NaOH trap and heated to reflux for 3 h. The solvent was removed by rotary evaporation and the residue washed with hexane to yield the aminoacid NCA as white residue.
LeuNCA: ¹H NMR (500 CDCl₃) 6.65 (s, 1H, NH), 4.33 (dd, 1H, α), 1.82 (m, 2H, β), 1.68 (m, 1H, γ), 0.98 (dd, 6H, δ).
PheNCA: ¹H NMR (500 CDCl₃) 7.36-7.18 (m, 5H), 5.84 (s, 1H, NH), 4.53 (dd, 1H), 3.28 (dd, 1H, α), 2.98 (dd, 1H, β).
Trp(Boc)NCA: : ¹H NMR (500 CDCl₃) 8.14 (d, 1H), 7.49 (d, 2H), 7.36 (t, 1H), 7.27 (m, 1H), 5.90 (s, 1H, NH), 4.59 (dd, 1H, α), 3.41 (dd, 1H, β), 3.07 (dd, 1H, β), 1.67 (s, 9H, t-Bu).
IleNCA: ¹H NMR (300 CDCl₃) 6.65 (s,1H, NH), 4.25 (d, 1H, α), 1.94 (m, 1H, β), 1.43 (dm, 2H, γ-CH₂), 1.43 (d, 3H, γ-CH₃), 0.94 (t, 3H, δ).
Lys(Boc)NCA: ¹H NMR (500 CDCl₃) 6.65 (bs, 1H, NₜH), 4.64 (s, 1H, carbamate NH), 4.31 (t, 1H, α), 3.13 (s, 2H, ε), 2.04 (m, 2H, β), 1.84 (m, 2H, δ), 1.48 (m, 11H, γ, t-Bu).
MetNCA: ¹H NMR (500 CDCl₃) 6.89 (s, 1H, NH), 4.50 (dd, 1H, α), 2.69 (t, 2H, γ), 2.14 (m, 1H, β), 2.08 (m, 4H, β, δ).

### Typical preparation of T4 N-capped homopolymers:

### T4-Leu₁₅

To IleNCA (0.200 g, 1.3 µmol) in 2.5 ml DMF was added isoleucine (0.012 g, 0.1 µmol). After stirring overnight under Ar T4-NCA (0.037 g, 0.050 µmol) was added and the reaction stirred an additional 72 h. The white solution was added to 8 ml H₂O. The heterogeneous solution was chilled to 4° C, centrifuged and the supernatant discarded and the pellet washed with 8 ml H₂O. The dried residue was washed with 50 ml ethanol warmed to 50° C to yield after drying, a white powder (0.124 g, 55%): ¹H NMR (500 DMSO) 7.75 (s, T4 aromatic), 7.08 (s, T4 aromatic), 4.11 (dd, α), 1.77 (m, β), 1.38 (m, β, γ-CH), 0.91 (m, γ-CH, γ-CH₃, δ).

### T4-Phe₁₅

White powder (58%); ¹H NMR (360 MHz, DMSO) 7.0-8.1 (NH, aromatics), 4.5 (α), 3.0 (β); MALDI-MS indicates T4-Phe₁₋₅.

### T4-Met₁₅

White powder (10%): ¹H NMR (500MHz, DMSO) 8.0-8.5 (amide NH), 4.4 (α) 2.5 (γ), 2.05 (ε), 2.0-1.7 (β).

### T4-Val₁₅

White powder (14%): ¹H NMR (500MHz, DMSO) 7.75 (T4 aromatic), 7.08 (T4 aromatic), 4.35 (α), 3.45 (β), 1.05 (γ).

For those conjugates that used a protected NCA an additional, separate deprotection step was necessary:
To T4-[Lys(Boc)]₁₅ (0.256 g, 61 µmol) in 10 ml of CH₂Cl₂ was stirred with trifluoroacetic acid (10 ml) for 2 h. The solvent was removed by rotary evaporation and the residue dissolved in 3 ml H₂O and ultrafiltered (Amicon regenerated cellulose, YM1, NMWL 1000, wash with 30 ml pH 5 H₂O). The retentate was dried in vacuum to give a light brown residue: ¹H NMR (500 D₂O) 7.82 (s, T4 aromatic), 7.41 (s, T4 aromatic), 4.29 (bs, α), 3.00 (bs, ε), 2.13-1.70 (m, β, δ, γ); MALDI-MS gives a range T4-LyS₄₋₁₁.
T4-Trp₁₅: ¹H NMR (500 DMSO) 8.25-6.80 (m, aromatic), 4.50 (bs, α), 3.40 (bs, β), 3.00 (bs, β).

### Typical preparation of T4 C-capped homopolymers:

To T4 (0.078 g, 100 µmol) in 10 ml dry DMF was added Trp(Boc)NCA (0.500 g, 1.514 mmol). After stirring for 64 h under Ar the reaction was quenched by adding 30 ml H2O. The cloudy white solution was chilled to 4° C, centrifuged and the pellet washed three times with 25 ml H2O. The residue was dried in vacuum to provide Trp(Boc)₁₅-T4 as a brown solid. This material was further purified by ultrafiltration (Amicon regenerated cellulose, YM1, NMWL 1000, wash with 30 ml pH 5 H₂O) to provide [Trp(Boc)]₁₅-T4 as a brown-gold solid (0.400 g, 79%): ¹H NMR (500 DMSO) 8.25-6.80 (m, aromatic), 4.50 (bs, α), 3.40 (bs, β), 3.00 (bs, β), 1.50 (bs, t-Bu).

To [Trp(Boc)]₁₅-T4 (0.509 g) in 8 ml of 1:1 CH₂Cl₂: trifluoroacetic acid was stirred for 1.5 h. The solvent was removed by rotary evaporation and the residue dried in vacuum to yield a brown solid (0.347 g, 97%): ¹H NMR (500 DMSO) 8.25-6.80 (m, aromatic), 4.50 (bs, β), 3.40 (bs, α), 3.00 (bs, β).
[Lys(Boc)]₁₅-T4: ¹H NMR (500 D₂O) 7.82 (s, T4 aromatic), 7.41 (s, T4 aromatic), 4.29 (bs, α), 3.00 (bs, ε), 2.13-1.70 (m, β, δ, γ).
Lys₁₅-T4: ¹H NMR (500 D₂O) 7.82 (s, T4 aromatic), 7.41 (s, T4 aromatic), 4.29 (bs, α), 3.00 (bs, ε), 2.13-1.70 (m, β, δ, γ).

### Typical preparation of random T4/homopolymers:

To T4NCA (0.065 g, 0.1 mmol) and Trp(Boc)NCA (0.400 g, 1.2 mmol) were combined in 4 ml dry DMF. Triethylamine (11 µl, 0.1 mmol) was added and the reaction stirred for 44 h under Ar. After quenching by the addition of 10 ml H₂O the heterogeneous mix was chilled to 4° C and centrifuged. The pellet was isolated and washed three times with 10 ml H₂O and dried in vacuum.

To the random T4/[Trp(Boc)]₁₅ polymer was added 10 ml 1:1 CH₂Cl₂: trifluoroacetic acid and the reaction stirred for 1 h. The solvent was removed by rotary evaporation to provide the deprotected polymer as a brown solid (0.262 g, 91%) which was further purified by ultrafiltration (Amicon regenerated cellulose, YM1, NMWL 1000, wash with 30 ml pH 5 H₂O): ¹H NMR (500 DMSO), 8.25-6.80 (m, aromatic), 4.50 (bs, α), 3.40 (bs, β), 3.00 (bs, β).
Random T4/Lys₁₅: ¹H NMR (500 D₂O); 7.82 (s, T4 aromatic), 7.41 (s, T4 aromatic), 4.29 (bs, α), 3.00 (bs, ε), 2.13-1.70 (m, β, δ, γ).

### Prparation of PolyLysine Depakote

To valproic acid (1.0 g, 6.9 mmol) in 14 ml 6:1 CH₂Cl₂:DMF was added N-hydroxysuccinimide (0.8 g, 6.9 mmol), dicyclohexylcarbodiimide (1.6 g, 7.6 mmol) and triethylamine (0.9 g, 8.9 mmol). The reaction was stirred for 60 h whereupon the solution was filtered to remove the white precipitate and the solvent removed by rotary evaporation. The residue was purified by flash chromatography (10:1-2:1 hexane:EtOAc) to provide the succinimidyl ester as a clear oil (1.0 g, 59%): R_{f} (3:1 hexane:EtOAc) 0.43; ¹H NMR (300 MHz, CDCl₃) 2.76 (s, 4H, succinimide), 2.61 (m, 1H, methine), 1.65-1.19 (m, 8H, methylene), 0.88 (t, 6H, methyl).
To Lys₁₄^{·}HBr (0.106 g, 37 µmol) in 0.8 ml H₂O pH 8 was added the valproic succinimidyl ester (0.104 g, 431 µmol) dissolved in 0.4 ml THF. The reaction was stirred overnight whereupon 8 ml H₂O was added. The mixture was acidified to pH 3 with 6 M HCl and extracted twice with 2 ml CH₂Cl₂. The aqueous layer was dried and the residue dissolved in 1 ml H₂O. The solution was purified by SEC (G-15,10 ml dry volume) and eluted with water. Those fractions containing conjugate were combined and dried to yield a white solid (0.176 mg) which by NMR indicated 28 Lysine for every one drug molecule; ¹H NMR (D₂O) 4.29 (m, 1H, α), 3.00 (m, 2H, ε), 1.87-1.68 (m, 4H, β, δ), 1.43 (m, γ, methylene), 0.85 (t, methyl).

### Preparation of PolyGlu Mevastatin

### AcNGlu₁₅(3-mevastatin)₂

To polyGlu₁₅ (0.11.6 g, 69 µmol) in 3 ml dry DMF was added 1 ml pyridine and acetic anhydride (20 µl, 207 µmol). After stirring for 21 h the mixture was acidified with 6 N HCl until pH 1 and then cooled to 4° C. The white precipitate was collected by centrifugation and washed three times with H₂O and then dried under vacuum to yield 11 mg of N-acetylated polyGlu₁₅.
To N-acetylated polyGlu₁₅ (0.011 g, 7 α mol) in 4.8 ml dry DMF was added dicyclohexylcarbodiimide (0.022 g, 108 µmol). After stirring twenty minutes the heterogeneous solution was filtered to remove insoluble dicyclohexylurea and combined with mevastatin (0.042 g, 108 µmol) and N-dimethyl-4-aminopyridine (0.013 g, 108 µmol). The mixture stirred for 23 h whereupon the reaction was quenched by the addition of 20 ml H₂O. The solution was extracted twice with 10 ml CHCl₃. The aqueous component was adjusted to pH 3 with 1 N HCl and cooled to 4° C. The resultant white precipitate was isolated by centrifugation and washed three times with 8 ml H₂O. The solid was dissolved in 1 ml H₂O and washed with 1 ml CH₂Cl₂ and twice with 2 ml EtOAc. The aqueous layer was acidified to pH 3 with 1. N HCl, cooled to 4° C, the precipitate isolated by centrifugation and washed twice with 2 ml H₂O. The dried conjugate (2 mg) was shown by ¹H NMR to contain fifteen Glu for every two mevastatin molecules: ¹H NMR (500 MHz, DMSO) 5.92 (5' mevastatin), 5.72 (3' mevastatin), 5.19 (4' mevastatin), 5.17 (8' mevastatin), 5.1.2 (3 mevastatin), 4.41 (5 mevastatin), 4.03 (α, Glu), 2.25 (γ, Glu), 1.88 (β, Glu), 0.82 (4",2' allylic methyl mevastatin), 1.17 (2" mevastatin).

### Glu₁₅(3-mevastatin) (160)

To Glu₁₅ (0.151 g, 77 µmol) in 3 ml dry DMF was added dicyclohexylcarbodiimide (0.239 g, 1.159 mmol) and the reaction stirred for 4 h under Ar. The white precipitate was removed and N-dimethyl-4-aminopyridme (0.141 g, 1.159 mmol) and mevastatin (0.222 g, 0.569 mmol) were added dissolved in 10 ml CHCl₃. The reaction stirred for 21 h under Ar whereupon the precipitate was removed. The solution was concentrated by rotary evaporation and added to 40 ml saturated NaCl (aq) adjusted so pH 8. The homogeneous solution was extracted three times with 20 ml CHCl3 and then ultrafiltered (Amicon regenerated cellulose, YM1, NMWL 1,000). The retentate was dried in vacuum to yield 8 mg of a white residue which showed a ratio of 15 Glutamic acids to one mevastatin by ¹H NMR (500 D₂O); 5.92 (5' mevastatin), 5.72 (3' mevastatin), 5.19 (4' mevastatin), 5.17 (8' mevastatin), 5.12 (3 mevastatin), 4.41 (5 mevastatin), 4.03 (α, Glu), 2.25 (γ, Glu), 1.88 (β, Glu), 0.82 (4",2' allylic methyl mevastatin), 1.17 (2" mevastatin).

### BocGlu(3-mevastatin)O-t-Bu

To BocGlu(OSu)O-t-Bu (0.181 g, 453 α mol) and mevastatin (0.177 g, 453 µmol) in 40 ml CHCl3 was added N-dimethyl-4-aminopyridine (0.055 g, 453 µmol). The reaction was heated to reflux for 7 h under Ar and then allowed to stir at 20° C for 8 h. The solvent was removed by rotary evaporation and the residue purified by flash chromatography (8:1-1:1 hexane:EtOAc) to provide the conjugate as a clear film (0.038 g, 11%): R_{f} (3:1 hexane:EtOAc) 0.22; ¹H NMR (CDCl₃ 500 MHz) 5.97 (d, 1H, 5'), 5.73 (dd, 1H, 3'), 5.55 (s, 1H, 4'), 5.32 (s, 1H, 8'), 5.24 (dd, 1H, 3), 5.09 (d, 1H, NH), 4.48 (m, 1H, 5), 4.20 (m, 1H, α), 2.78 (m, 2H, 2), 2.37 (m. 4H, 2', 2", γ), 1.45 (s, 18H, t-Bu), 1.12 (d, 3H, 2"-CH₃), 0.88 (m, 6H, 4", 2'-CH₃).

### Preparation of PolyGlu Prednisone

### BocGlu(21-Prednisone)O-t-Bu

To BocGlu-O-t-Bu (0.400 g, 1.32 mmol) in 20 ml CHCl3 was added dicyclohexylcarbodiimide (0.544 g, 2.64 mmol). The reaction was stirred for 1 h and filtered to remove insoluble dicyclohexylurea. N-dimethyl-4-aminopyridine (0.320 g, 2.64 mmol) and prednisone (0.472 g, 1.32 mmol) was added. The reaction was stirred for 60 h and filtered. The solvent was removed by rotary evaporation and the residue purified by flash chromatography (10:1-0:1 hexane:EtOAc) to provide the target as a clear film (0.256 g, 31%): R_{f} (6:1 CHCl₃:MeOH) 0.54; ¹H NMR (CDCl₃ 500 MHz) 7.68 (d, 1H, 1), 6.16 (d, 1H, 2), 6.04 (s, 1H, 4), 5.15 (d, 1H, NH), 5.03 (d, 1H, 21), 4.71 (d, 1H, 21), 4.08 (t, 1H, α), 1.40 (s, 18H, t-Bu).

### Glu(21-Prednisone)

To BocGlu(21-Prednisone)O-t-Bu (0.060 g, 93 µmol) in 15 ml CH₂Cl₂ was stirred for 1 h with trifluoroacetic acid (1.5 ml). The solvent was removed by rotary evaporation and the residue purified by flash chromatography (8:1 CHCl₃:MeOH) to yield a clear film: R_{f} (6:1 CHCl₃:MeOH) 0.13 ¹H NMR (CDCl₃ 500 MHz) 7.72 (d, 1H, 1), 6.25 (d, 1H, 2), 6.14 (s, 1H, 4), 5.14 (d, 1H, 21), 4.75 (d, 1H, 21), 4.10 (t, 1H, α).

### EXAMPLE 19

### Amine-Initiated Polymerization of L-Glutamic Acid NCA

The following procedure was successfully used to synthesize the polyglutamic acid conjugate of atenolol. 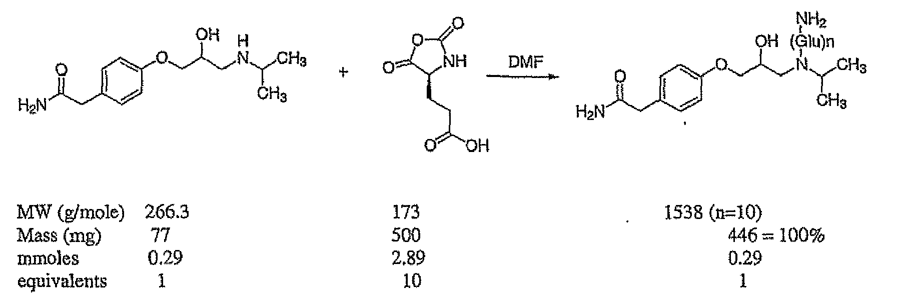 DMF is dimethylformamide, anhydrous, and was purchased from Aldrich. Glassware was oven-dried prior to use.
1. Glu-NCA (500 mg, 2.89 mmoles) was dissolved in 4 mL of DMF and stirred under Ar in a 15 mL roundbottom flask equipped with a gas inlet tube.
2. Atenolol, dissolved in 1 mL of DMF, was added to this solution of Glu-NCA and allowed to stir at room temperature for 72 h. In general, the reactions can be run until there is no free amine initiator by tlc. For this reaction, tlc was run using silica plates and eluting with 20% methanol in ethyl acetate.
3. The reaction was quenched by pouring into 20 mL of 10% sodium bicarbonate in water (pH = 8).
4. The water was washed with 3 X 20 mL of methylene chloride and 3 X 20 mL of ethyl acetate.
5. Combined aqueous layers were brought to a pH of 6 with 6N HCl and reduced to a volume of about 20 mL by rotary evaporation. This solution was then cooled in the refrigerator for > 3 hours.
6. To precipitate the polymeric product, the aqueous solution was then acidified to a pH of about 2 using 6N HCl and placed back in the refrigerator for 1-2 hours.
7. The suspension was poured by portions into a 10 mL test tube and centrifuged for 15 minutes until the precipitate formed a solid pack at the bottom of the tube from which the water could be decanted. (At this point in the general procedure, it is preferable that the solid be filtered through a filter funnel and washed with acidic water. The centrifuge was used for atenolol because the solid was too thin to filter.)
8. The solid was then resuspended in acidic water (pH about 2) and vortexed before being centrifuged again and the water decanted. This procedure was repeated once more for a total of three washes.
9. The solid was then dried by high vacuum overnight yielding 262 mg (59%) of polymer. NMR analysis indicated that the Glu/Atenolol ratio was about 30/1.

### EXAMPLE 20

Monolayers of Caco-2 human intestinal epithelial cells are increasingly being used to predict the absorption of orally delivered drugs. We used the Caco-2 transwell system and other *in vitro* assays to evaluate the performance of Polythroid. Our findings indicate that Polythroid may enhance oral delivery of thyroid hormones for the treatment of hypothyroid disorders.

### IN VITRO PERFORMANCE

### Caco-2 human intestinal epithelial cell assay

Caco-2 cells are grown on the surface of collagen coated wells in a 24 well format to form confluent monolayers that represent small segments of the intestine. The wells are removable and contain a top chamber representing the apical side (facing the lumen of the intestine) and a bottom chamber representing the basolateral side (site of serosal drug absorption). The integrity of the epithelial barrier is monitored by testing the electrical resistance across the monolayer. Absorption of drugs can be studied by adding sample to the apical side and assaying the concentration of the drug in the basolateral chamber following incubation.

### Intestinal epithelial cell proteases digest Polythroid

Polythroid is a synthetic polymer of glutamic acid with T4 and T3 covalently attached by a peptide bond linkage. The polymer is the delivery vehicle for the thyroid hormones and is not designed to cross the intestinal barrier itself. Rather, it is designed to release T4 and T3 in a time dependent manner. Release of the thyroid hormones is dependent on the enzymatic cleavage of the glutamic acid polymer. In theory, this will result from Polythroid encountering proteolytic enzymes as it descends the intestinal tract. Proteins are digested into small polypeptides by gastric pepsin and pancreatic enzymes secreted into the small intestine. Intestinal epithelial cells then function to further breakdown the small polypeptides. They accomplish this with proteolytic enzymes referred to as brush border proteases that are attached to the cell surface.

Monitoring the effect of brush border peptidases on Polythroid required development of an assay to specifically distinguish Polythroid from polyglutamic acid and the thyroid hormones. Therefore, we developed an enzyme-linked immunosorbent assay (ELISA) that specifically recognizes Polythroid. The assay employs antibodies against the glutamic acid polymer to capture Polythroid and antibodies to T4 or T3 to detect the presence of Polythroid. The assay has no cross-reactivity with polyglutamic acid or the thyroid hormones themselves. Consequently, proteolytic degradation of Polythroid results in T4 and T3 release from the polymer and a corresponding decrease in ELISA reactivity. The Polythroid specific ELISA can, therefore, be used to monitor the breakdown of Polythroid.

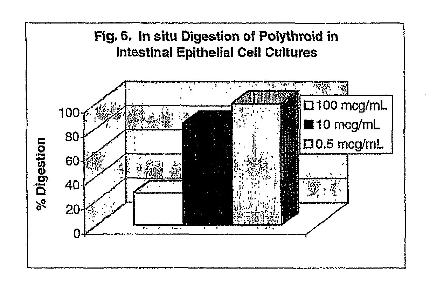

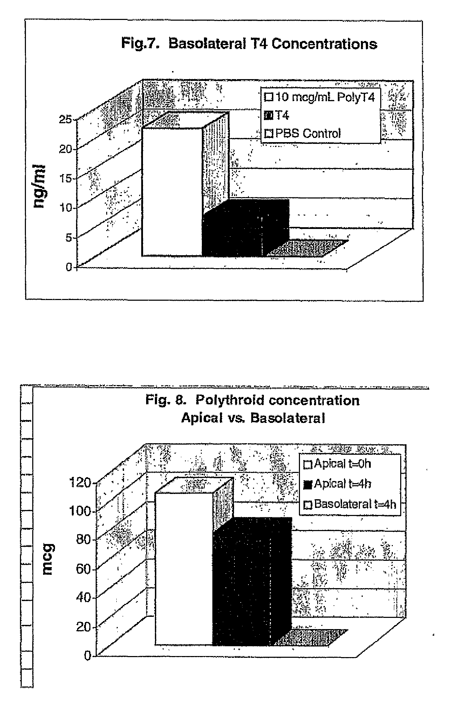

The Polythroid specific assay was used to analyze *in situ* digestion of Polythroid in Caco-2 cell cultures. Different concentrations of Polythroid were added to the apical side of Caco-2 cells and incubated for 4 hours in PBS at 37°C (n=4). The apical side Polythroid concentration was measured by Polythroid specific ELISA before and after the 4 hour incubation (Fig. 6). At the relatively high concentration of 100 micrograms, 26% of Polythroid was degraded, whereas at a 10-fold lower concentration 84% of the Polythroid was degraded. When a concentration of 0.5 micrograms was added (closer to the concentrations that would be encountered by the intestine in a normal human dose) the amount of Polythroid remaining after 4 hours of incubation was below the limit of detection for the ELISA (10 ng) indicating essentially complete digestion. The loss of Polymer in the apical chamber was not due to absorption of Polythroid across the monolayer since the basolateral chamber contained no detectable Polythroid in any of the experiments (see below). We cannot rule out cellular uptake of Polythroid, however, enzymatic digestion is likely to account for most, if not all, of the decrease in Polythroid concentration on the apical side. At the higher concentrations, it would be difficult for cellular uptake to account for such a large difference in the remaining Polythroid.
Polythroid enhances absorption of T4 across Caco-2 monolayers **Absorption of T4 was monitored in the Caco-2 transwell system (n=4). Polythroid (10 micrograms) was added to the apical side of the transwells. T4 was added to the apical side at a concentration equal to the T4 content of Polythroid. A commercial ELISA for T4 was used to determine the level of T4 in the basolateral chamber following incubation for 4 hours at 37°C (****Fig. 7****). A significantly higher amount of T4 was absorbed from Polythroid as compared to CaCo-2 cells incubated with the amount of T4 equivalent to that contained in the polymer.**

### Polythroid does not cross Caco-2 monolayers

In order to determine if Polythroid itself crosses the Caco-2 monolayer we used the Polythroid specific ELISA to measure the amount of polymer in the basolateral chamber after incubation with Polythroid at a high concentration (100 micrograms). After 4 hours incubation, samples (n=4) from the basolateral side showed no reactivity in the ELISA (Fig. 8). The limit of detection for Polythroid is 10 ng, therefore, less than 1/10,000 of the Polythroid was absorbed. In conclusion, within the limits of ELISA detection, Polythroid does not cross the Caco-2 monolayer.

### Digestion of Polythroid in gastric and intestinal simulators

Pepsin secreted by the gastric mucosa is the only protease active in the acid conditions of the stomach. The pancreas secretes a number of proteolytic enzymes into the intestine which degrade proteins and polypeptides. In theory, these endogenous proteases will participate in release of T4 and T3 from Polythroid as the polymer descends the intestinal tract.

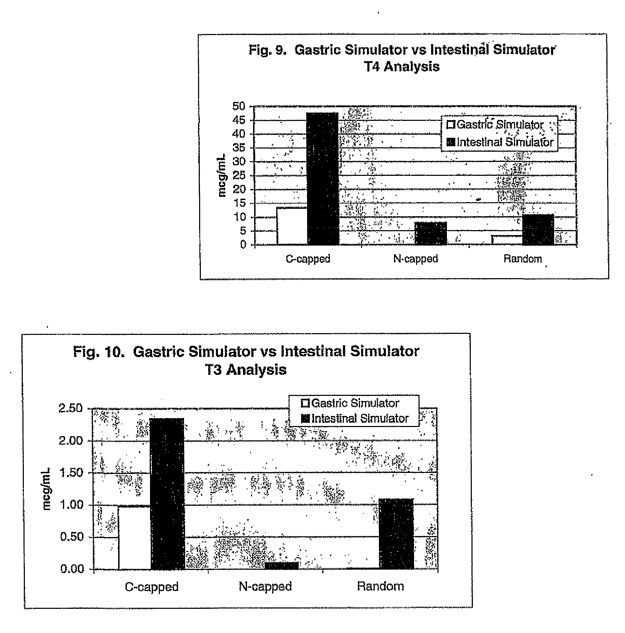

We tested Polythroid in the USP gastric simulator and the USP intestinal simulator and compared the levels of digestion for Polythroid synthesized by different methods. The samples of Polythroid varied in the position of thyroid hormone attachment. Samples were dissolved in gastric simulator buffer containing pepsin or in intestinal simulator buffer containing pancreatic enzyme extract (pancreatic) and incubated for 24 hours at 37°C. Following digestion, samples were analyzed by HPLC for the content of released monomeric T4 and T3. Figures 9 and 10 show the levels of T4 and T3 following digestion in the gastric and intestinal simulators. Release varied depending on the position of thyroid hormone attachment. Polythroid with T4 and T3 attached at the C-terminus (C-capped) showed the highest level of digestion. On the other hand, Polythroid with N-terminal attachment (N-capped) showed no digestion in the gastric simulator and a relatively low amount of digestion in the intestinal simulator. Polythroid with random attachment showed only marginal digestion in the gastric simulator and moderate digestion in the intestinal simulator. In conclusion, the rate of thyroid hormone release from Polythroid varies depending on the method of synthesis. This provides a potential means of controlling (fine tuning) time release of oral delivery.

### Conclusions and Summary

The following conclusions can be drawn from *in vitro* performance assays:
- T4 and T3 are released from Polythroid by pancreatic and intestinal cell proteases
- T4 and T3 released from Polythroid are absorbed across intestinal monolayers
- Polythroid enhances absorption of T4 across intestinal epithelium *in vitro*
- Polythroid itself does not cross the intestinal epithelial barrier *in vitro*
- The kinetics of time release may be controlled by the method of Polythroid synthesis

Covalent attachment of T4 and T3 to a polypeptide affords a slumber of potential advantages to oral delivery for thyroid hormone replacement therapy. Proteolytic enzymes produced by the pancreas and intestinal epithelial cells release T4 and T3 from Polythroid. Therefore, T4 and T3 should be released in a time dependent manner as they descend the intestinal tract. Once released the hormones are absorbed across the intestinal epithelium in the Caco-2 cell model. In addition, data from the *in vitro* intestinal epithelial model suggests that attachment of T4 to polymers of glutamic acid may enhance absorption of the thyroid hormones, perhaps by providing a second mechanism of uptake and/or enhancing solubility of the hormones. Polythroid itself does not cross the intestinal epithelial barrier in the *in vitro* Caco-2 model. Thus, any concerns about systemic effects of the polymer are minimized since it should not be absorbed into the bloodstream.

Although illustrated and described above with reference to specific embodiments, the invention is nevertheless not intended to be limited to the details shown. Rather, various modifications may be made in the details within the scope and range of equivalents of the claims and without departing from the spirit of the invention.

## Claims

1. A composition comprising:
a polypeptide; and
an active agent selected from the group comprising:
abacavir sulfate, abarelix, acarbose, Acetaminophen, Acetaminophen; Codeine phosphate, Acetaminophen; Propoxyphene napsylate, Acetylsalicylic acid, Acitretin, activated protein C, Acyclovir, adefovir dipivoxil, adenosine, Adrenocorticotrophic hormone, Albuterol, alendronate sodium, Allopurinal, alpha 1 proteinase inhibitor, Alprazalom, alprostadil, altinicline, amifostine, Amiodarone, Amitriptyline HCL, amlodipine besylate, amlodipine besylate ; benazepril hcl, Amoxicillin, amoxicillin ; clavulanate potassium amprenavir, anagrelide hydrochloride, anaritide, anastrozole, antisense oligonucleotide, aripiprazole, Astemizol, Atenolol, atorvastatin calcium, atovaquone, avasimibe, Azathioprine, azelastine hydrochloride, Azithromycin dihydrate, Baclofen, befloxatone, benazepril hydrochloride, Benzatropine Mesylate, Betamethasone, bicalutamide, Bisoprolol/Hydrochlorothiazide, bosentan, Bromocriptine, Bupropion hydrochloride, Buspirone, Butorphanol tartrate, cabergoline, caffiene, calcitriol, candesartan cilexetil, candoxatril, capecitabine, Captopril, carbamazepine, Carbidopa/Levodopa, carboplatin, Carisoprodol, carvedilol, caspofungin, Cefaclor, Cefadroxil; Cefadroxil hemihydrate, Cefazolin sodium, Cefdinir, Cefixime, 1555; 1555U88, Cefotaxime sodium, Cefotetan disodium, Cefoxitin sodium, Cefpodoxime proxetil, Cefprozil, Ceftazidime, Ceftibuten dihydrate, 264W94, Cefuroxime axetil, Cefuroxime sodium, celecoxib, Cephalexin, cerivastatin sodium, cetirizine hydrochloride, Chlorazepate Depot, Chlordiazepoxide, ciclesonide, cilansetron, Cilastatin sodium; lmipenem, cilomilast, Cimetidine, ciprofloxacin, cisapride, cisatracurium besylate, cisplatin, citalopram hydrobromide, clarithromycin, Clomipramine, Clonazepam, Clonidine HCL, clopidogrel bisulfate, 4030W92, clorpheniramine tannate, Clozapine, Colestipol HCL, conivaptan, Cyclobenzaprine HCL, Cyclophosphamide, Cyclosporine, dalteparin sodium, dapitant, desmopressin acetate, Desogestrel ; ethinyl estradiol, Dextroamphetamine sulfate, dextromethorphan, Diazepam, ABT 594, Diclofenac sodium, diclofenac sodium, misoprostol, Dicyclomine HCL, didanosine, Digoxin, diltiazem hydrochloride, Dipyridamole, divalproex sodium, d-methylphenidate, dolasetron mesylate monohydrate, donepezil hydrochloride, Dopamine/D5W, Doxazosin, doxorubicin hydrochloride, duloxetine, dutasteride, ecadotril, ecopipam, edodekin alfa (lnterleukin-12), efavirenz, ABT 773, emivirine, Enalapril, enapril maleate, hydrochlorothiazide, eniluracil, enoxaparin sodium, epoetin alfa recombinant, eptifibatide, Ergotamine Tartrate, Erythromycin, ALT 711, esatenolol, Esterified estrogens; Methyltestosterone, Estrogens, conjugated, Estrogens, conjugated; medroxyprogesterone acetate, Estropipate, etanercept, ethinyl estradiol/norethindrone, BMS CW 189921, Ethinyl estradiol ; Ethynodiol diacetate, Ethinyl estradiol; Levonorgestrel, Ethinyl estradiol; Norethindrone, Ethinyl estradiol; Norethindrone acetate, Ethinyl estradiol; Norgestimate, Ethinyl estradiol; Norgestrel, Etidronate disodium, Etodolac, Etoposide, etoricoxib, exendin-4, famciclovir, Famotidine, Felodipine, fenofibrate, fenretinide, Fentanyl, fexofenadine hydrochloride, filgrastim SD01, finasteride, flecainide acetate, fluconazole, Fludrocortisone acetate, flumazenil, Fluoxetine, Flutamide, fluvastatin, Fluvoxamine maleate, follitropin alfa/beta, Formoterol, Fosinopril, fosphenytoin sodium, Furosemide, Gabapentin, gadodiamide, gadopentetate dimeglumine, gadoteridol, ganaxolone, ganciclovir, gantofiban, gastrin CW17 immunogen, gemcitabine hydrochloride, Gemfibrozil, Gentamicin lsoton, gepirone hydrochloride, glatiramer acetate, glimepiride, Glipizide, Glucagon HCL, Glyburide, granisetron hydrochloride, Haloperidal, BMS 284756, Hydrochlorothiazid, Hydrochlorothiazide; Triamterene, Hydromorphone HCL, Hydroxychloroquine sulfate, lbuprofen, Idarubicin HCL, ilodecakin, ilomastat, imiglucerase, lmipramine HCL, indinavir sulfate, infliximab, insulin lispro, interferon alfacon-1, interferon beta-1a, interleukin-2, iodixanol, iopromide, loxaglate meglumine ; loxaglate sodium, lpratropium, Irbesartan, irinotecan hydrochloride, lsosorbide Dinitrate, lsotretinoin, lsradipine, itasetron, ltraconazole, Ketoconazole, Ketoprofen, Ketorolac, Ketotifen, Labetalol HCL, lamivudine, lamivudine; zidovudine, lamotrigine, lansoprazole, lansoprazole, amoxicillin, clarithromycin, leflunomide, lesopitron, Leuprolide acetate, levocarnitine, levocetirizine, Levofloxacin, Levothyroxine, lintuzumab, Lisinopril, lisinopril; hydrochlorothiazide, CS 834, Loperamide HCL, Loracarbef, loratadine, Lorazepam, losartan potassium, losartan potassium; hydrochlorothiazide, Lovastatin, marimastat, mecasermin, Medroxyprogesterone Acetate, mefloquine hydrochloride, megestrol acetate, CVT CW 124, Mercaptopurine, Meropenem, mesalamine, mesna, Metaxalone, Metfomin, EM 800, Methylphenidate HCL, Methylprednisolone Acetate, FK 463, Metolazone, metoprolol succinate, MK 826, Metronidazole, milrinone lactate, Minocycline HCL, mirtazapine, Misoprostol, mitiglinide, mitoxantrone hydrochloride, mivacurium chloride, modafinil, moexepril hydrochloride, montelukast sodium, Morphine Sulfate, Mycophenolate mofetil, nabumetone, Nadolol, Naproxen sodium, naratriptan hydrochloride, nefazodone hydrochloride, nelarabine, nelfinavir mesylate, nesiritide, nevirapine, nifedipine, nimodipine, nisoldipine, nitrofurantoin, nitrofurantoin, macrocrystalline, Nitroglycerin, nizatidine, norastemizole, Norethindrone, norfloxacin, Nortriptyline HCL, octreotide acetate, Oxycodone/APAP, oflaxacin, olanzapine, Omeprazole, ondansetron hydrochloride, oprelvekin, orlistat, Orphenadrine citrate, Oxaprozin, Oxazepam, oxybutynin chloride, Oxycodone HCL, GM 611, M-CSF, pagoclone, palivizumab, pamidronate disodium, paricalcitrol, paroxetine hydrochloride, pemetrexed, Pemoline, penicillin V, pentosan polysulfate sodium, Pentoxifylline, Pergolide, NE 0080, Phenobarbital, Phenytoin sodium, pioglitazone hydrochloride, Piperacillin sodium, pleconaril, poloxamer CW 188, posaconazole, NN 304, pramipexole dihydrochloride, pravastatin sodium, Prednisone, pregabalin, Primidone, prinomastat, Prochlorperazine maleate, Promethazine HCL, PD 135158, Propoxyphene-N/APAP, Propranolol HCL, prourokinase, quetiapine fumarate, quinapril hydrochloride, rabeprazole sodium, raloxifine hydrochloride, Ramipril, Ranitidine, ranolazine hydrochloride, relaxin, remacemide, repaglinide, repinotan, ribavirin+peginterferon alfa-2b, riluzole, Rimantadine HCL, risperidone, ritonavir, rizatriptan benxoate, rocuronium bromide, rofecoxib, ropinirole hydrochloride, rosiglitazone maleate, Goserelin, rubitecan, sagramostim, saquinavir, Docetaxel, satraplatin, Selegiline HCL, sertraline hydrochloride, sevelamer hydrochloride, sevirumab, sibutramine hydrochloride, sildenafil citrate, simvastatin, sinapultide, sitafloxacin, sodium polystyrene sulfonate, Sotalol HCL, sparfosic acid, Spironolactone, stavudine, sucralfate, sumatriptan, tabimorelin, tamoxifen citrate, tamsulosin hydrochloride, Temazepam, tenofovir disoproxil, tepoxalin, Terazosin HCL, terbinafine hydrochloride, terbutaline sulfate, teriparatide, tetracycline, thalidomide, Theophylline, Thiotepa, thrombopoetin, TPO, tiagabine hydrochloride, ticlopidine hydrochloride, tifacogin, tirapazamine, tirofiban hydrochloride, tizanidine hydrochloride. Tobramycin sulfate, tolterodine tartrate, tomoxetine, topiramate, Topotecan HCL, toresemide, tPA analogue, Tramadol HCL, trandolapril, trastuzumab, Trazadone HCL, Triamterene/HCTZ, troglitazone, trovafloxacin mesylate, urokinase, Ursodiol, valacyclovir hydrochloride, valdecoxib, Valproic Acid, valsartan, hydrochlorothiazide, valspodar, Vancomycin HCL, Vecuronium bromide, venlafaxine hydrochloride, Verapamil HCL, vinorelbine tartrate, Vitamin B12, Vitamin C, voriconazole, Warfarin Sodium, xaliproden, zafirlukast, zaleplon, zenarestat, zidovudine, zolmitriptan, Zolpidem, bleomycin, Phytoseterol, paclitaxel, Flutiasone, Fluorouracil, Pseudoephedrine, A 78773, AGl 1067, BCX CW 1812, BMS CW 188667, BMS CW 193884, BMS CW204352, BPI 21, CD11a, CEB 925, Propofol, GT 102279, Recombinant hepatitis vaccine, L 159282, LFA3TIP, Daily Multi Vit, Erythromycn/Sulfsx, Ethinyl estradiol ; Desogestrel, Lithium Carbonate, LYM 1, Methylprednisolone, Sodium succinate, rotavirus vaccine, saquinavir mesylate, arginine, heparin, Thymosin alpha, montelukast sodium and fexofenadine hydrochloride, lodothyronine, lodothyronine and thyroxine, Codeine, Ethylmorphine, Diacetylmorphine, Hydromorphone, Hydrocodone, Oxymorphone, Dihydrocodeine, Dihydromorphine, Methyldihydromorphinone, Codeine and promethazine, Codeine, phenylephrine and promethazine, Codeine and guaifenesin, Codeine, guaifenesin and pseudoephedrine, Aspirin, carisoprodol and codeine, Himatropine methylbromide and hydrocodone bitartrate, Hydrocodone bitartrate and phenylpropanolamine, Acetaminophen and hydrocodone bitartrate, Chlorpheniramine maleate, hydrocodone bitartrate and pseudoephedrine, Guaifenesin and hydrocodone, lbuprofen and hydrocodone, Chlorpheniramine polistirex and hydrocodone polystirex, naltrexone.
covalently attached to said polypeptide.

2. The composition of claim 1 wherein said active agent is selected from the group comprising: Butorphanol tartrate, Dextroamphetamine sulfate, dextromethorphan, Hydromorhone HCL, Methylphenidate HCL, Morphine Sulfate, Naproxen sodium, Oxycodone/APAP, Oxycodone HCL, Pseudoephidrine, Codeine, Ethylmorphine, Diacetylmorphine, Hydromorphone, Hydrocodone, Oxymorphone, Dihydrocodeine, Dihydromorphine, Methyldihydromorphinone, Codeine and promethazine, Codeine, phenylephrine and promethazine, Codeine and guaifenesin, Codeine, guaifenesin and pseudoephedrine, Aspirin, carisoprodol and codeine, Himatropine methylbromide and hydrocodone bitartrate, Hydrocodone bitartrate and phenylpropanolamine, Acetaminophen and hydrocodone bitartrate, Chlorpheniramine maleate, hydrocodone bitartrate and pseudoephedrine, Guaifenesin and hydrocodone, lbuprofen and hydrocodone, Chlorpheniramine polistirex and hydrocodone polystirex naltrexone.

3. The composition of claim 1 wherein said polypeptide is a homopolymer of a naturally occurring amino acid.

4. The composition of claim 1 wherein said polypeptide is a heteropolymer of two or more naturally occurring amino acids.

5. The composition of claim 1 wherein said polypeptide is a homopolymer of a synthetic amino acid.

6. The composition of claim 1 wherein said polypeptide is a heteropolymer of two or more synthetic amino acids.

7. The composition of claim 1 wherein said polypeptide is a heteropolymer of one or more naturally occurring amino acids and one or more synthetic amino acids.

8. The composition of claim 1 wherein said active agent is covalently attached to a side chain, the N-terminus or the C-terminus of said polypeptide.

9. The composition of claim 1 wherein said active agent is a carboxylic acid and wherein said active agent is covalently attached to the N-terminus of said polypeptide.

10. The composition of claim 1 wherein said active agent is an amine and wherein said active agent is covalently attached to the C-terminus of said polypeptide, or said active agent is an alcohol and wherein said active agent is covalently attached to the C-terminus of said polypeptide, or said active agent is an alcohol and wherein said active agent is covalently attached to the N-terminus of said polypeptide.

11. The composition of claim 1 further comprising a microencapsulating agent.

12. The composition of claim 13 wherein said microencapsulating agent is selected from the group consisting of polyethylene glycol (PEG), an amino acid, a sugar and a salt.

13. The composition of claim 1 further comprising an adjuvant.

14. The composition of claim 1 wherein said composition is in the form of an ingestable tablet, or is in the form of an intravenous preparation, or is in the form of an oral suspension.

15. A method for protecting an active agent as defined in any preceding claim from degradation comprising covalently attaching said active agent to a polypeptide.

16. A method for controlling release of an active agent as defined in any preceding claim from a composition wherein said composition comprises a polypeptide, said method comprising covalently attaching said active agent to said polypeptide.

17. A method for delivering an active agent to a patient comprising administering to said patient a composition comprising:
a polypeptide; and
an active agent as defined in any preceding claim covalently attached to said polypeptide.

18. A method for preparing a composition comprising a polypeptide and an active agent as defined in any preceding claim covalently attached to said polypeptide, said method comprising the steps of:
(a) attaching the active agent to a side chain of an amino acid to form an active agent/amino acid complex;
(b) forming an active agent/amino acid complex N-carboxyanhydride (NCA) from said active agent/amino acid complex; and
(c) polymerizing said active agent/amino acid complex N-carboxyanhydride (NCA).

19. The method of claim 18 wherein steps (a) and (b) are repeated prior to step (c) with a second active agent.

20. The method of claim 18 wherein said active agent and said second active agent are copolymerized in step (c).

21. The method of claim 18 wherein said amino acid is glutamic acid and wherein said active agent is released from said glutamic acid as a dimer upon a hydrolysis of the polypeptide and wherein said active agent is released from said glutamic acid by coincident intramolecular transamination.

22. The method of claim 21 wherein said glutamic acid is replaced by an amino acid selected from the group consisting of aspartic acid, arginine, asparagine, cysteine, lysine, threonine, and serine, and wherein said active agent is attached to the side chain of the amino acid to form an amide, a thioester, an ester, an ether, a urethane, a carbonate, an anhydride or a carbamate.

23. The method of claim 22 wherein said glutamic acid is replaced by a synthetic amino acid with a pendant group comprising an amine, an alcohol, a sulfhydryl, an amide, a urea, or an acid functionality.
